# EUROPEAN PATENT APPLICATION

(11) **EP 2 722 000 A1**
(43) Date of publication of application: **23.04.2014**
(21) Application number: 14151268.1
(22) Date of filing: 04.02.2009
(51) Int. Cl.: A61B 5/04

(54) **System and devive for locating the tip of a central venous catheter**

(30) Priority: 05.02.2008 US 26372
(62) Divisional of application: 09707467.8
(71) Applicant: Rothenberg, Peter M., Laguna Niguel, California 92677 (US); Bard Access Systems, Inc., Salt Lake City, UT 84116 (US)
(72) Inventor: Rothenberg, Peter M., Laguna Niguel, CA California 92677 (US)
(74) Representative: HOFFMANN EITLE

(57) **Abstract**

Methods of locating a tip of a central venous catheter ('CVC') relative to the superior vena cava, sino-atrial node, right atrium, and/or right ventricle using electrocardiogram data. The CVC includes at least one electrode. In particular embodiments, the CVC includes two or three pairs of electrodes. Further, depending upon the embodiment implemented, one or more electrodes may be attached to the patient's skin. The voltage across the electrodes is used to generate a P wave. A reference deflection value is determined for the P wave detected when the tip is within the proximal superior vena cava. Then, the tip is advanced and a new deflection value determined. A ratio of the new and reference deflection values is used to determine a tip location. The ratio may be used to instruct a user to advance or withdraw the tip.

## Description

### CROSS REFERENCE TO RELATED APPLICATION(S)

This application claims the benefit of United States Provisional Application Serial No. 61/026,372, filed February 5, 2008.

### BACKGROUND OF THE INVENTION

### Field of the Invention

The present invention is directed generally to devices for and methods of locating a catheter inside a body and more particularly to devices for and methods of locating the tip of a central venous catheter inside the superior vena cava, right atrium, and/or right ventricle using information obtained from an electrocardiogram.

### Description of the Related Art

Central venous catheters ("CVC") include any catheter designed to utilize the central veins (e.g., subclavian and superior vena cava) or right sided cardiac chambers for the delivery and/or withdrawal of blood, blood products, therapeutic agents, and/or diagnostic agents. CVCs also include catheters inserted into the central veins or right sided cardiac chambers for the acquisition of hemodynamic data. Standard central venous catheters for intravenous access, dialysis catheters, percutaneously introduced central catheters ("PICC" lines), and right heart ("Swan-Ganz™") catheters are examples of CVCs.

The standard of care for placing a CVC (other than right heart catheters which generally terminate in the pulmonary artery) dictates that the tip of the CVC lie just above and not inside the right atrium. In fact, in 1989, the Food and Drug Administration issued a warning citing an increased risk of perforation of the right atrium, clot formation, and arrhythmias among other potential complications resulting from the tip of the CVC being placed inside the right atrium.

While CVCs have been used for many years, determining the position of the tip of the CVC has always been problematic. Currently, a chest x-ray is used to determine the position of the tip of the CVC. Because CVC may be a radiopaque and/or include radiopaque materials, the CVC is visible on an x-ray. However, this method has several drawbacks. For example, obtaining a chest x-ray is labor intensive and expensive. In recent years, CVCs, which were traditionally placed in a hospital in-patient setting, are being placed in an outpatient setting more frequently. In an outpatient setting, obtaining a chest x-ray to determine the position of the tip of the CVC can be very cumbersome and may not be obtained in a timely manner. Therefore, using a chest x-ray to determine the position of the tip of the CVC may introduce a considerable delay, prolonging the procedure. Generally, the operator will leave the patient to perform other duties while the x-ray is processed. If the tip is improperly placed, the operator must return to the patient's bedside to reposition the CVC. To reposition the CVC, the operator must open the sterile dressing, cut the sutures, re-suture, and redress the wound, all of which potentially expose the patient to discomfort and infection.

Recently, navigational systems principally used to guide peripherally placed lines have become available. Based upon the detection of magnetic fields between a stylet tip and a detector, these systems assume (and depend upon) a relationship between surface landmarks and anatomic locations. Unfortunately, these systems cannot be used to determine the location of the tip of a CVC with sufficient accuracy because the relationship between surface landmarks and anatomic locations is highly variable from one patient to another.

In addition to the need to know where the tip is during initial placement, the CVC may migrate or otherwise move after the initial placement and require re-positioning. Therefore, the operator must monitor or periodically reevaluate the location of the tip.

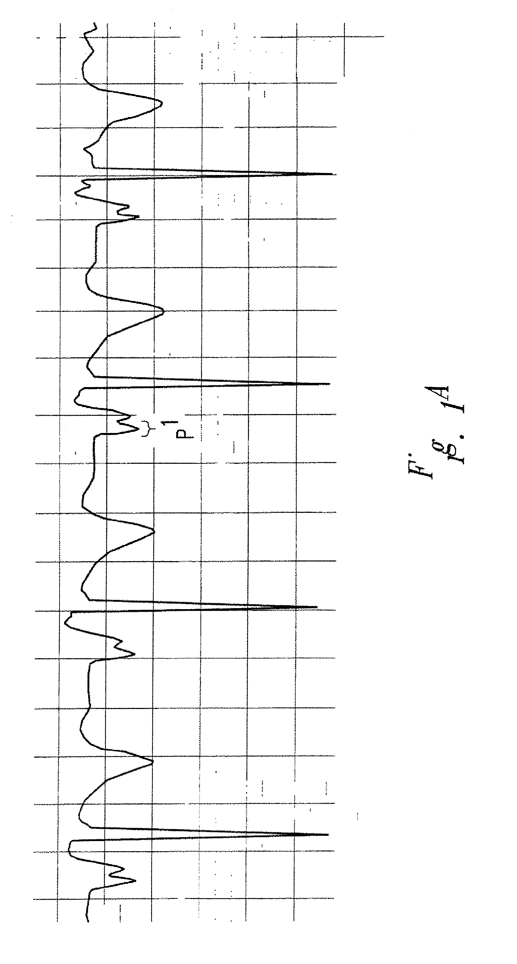

An electrocardiogram ("ECG") measures electrical potential changes occurring in the heart. Referring to Figures 1A-1C, the ECG measurements may be visualized or displayed as an EGG trace, which includes ECG waveforms. As is appreciated by those of ordinary skill in the art, ECG waveforms are divided into portions that include a QRS complex portion and a P wave portion in addition to other wave portions. The QRS complex corresponds to the depolarization of the ventricular muscle. The P wave portion of the ECG waveforms represents atrial muscle depolarization: the first half is attributable to the right atrium and the second half to the left atrium. Under normal circumstances, atrial muscle depolarization is initiated by a release of an excitatory signal from the sino-atrial ("SA") node, a specialized strip of tissue located at the juncture of the superior vena cava ("SVC") and right atrium.

As is appreciated by those of ordinary skill in the art, an ECG may be obtained using different electrode configurations. For example, a standard configuration referred to as "Lead II" may used. In a bipolar Lead II configuration, one of the electrodes (the cathode) is attached to the left leg and the other electrode (the anode) is attached to the right shoulder. As is appreciated by those of ordinary skill in the art, using a different configuration could change the polarity and/or the shape of the P wave. Other standard bipolar configurations include a bipolar Lead I configuration where the cathode is attached to the left shoulder and the anode is attached to the right shoulder and a bipolar Lead III configuration where the cathode is attached to the left leg and the anode is attached to the right shoulder.

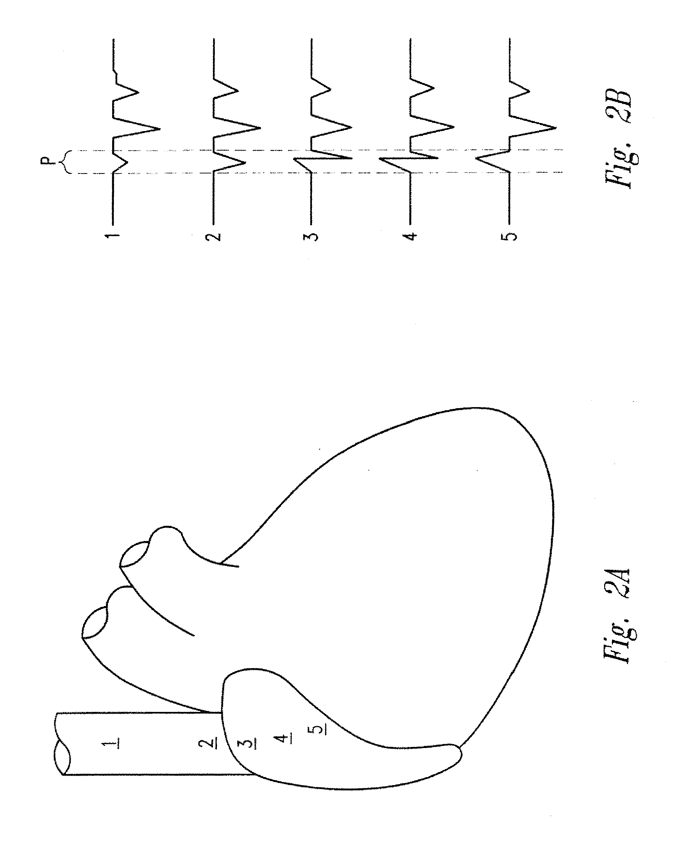

The waveforms depicted in Figures 1A-1C and 2B were obtained using the anode of a standardized bipolar ECG Lead II configuration attached to the right shoulder and the tip of the CVC as the cathode. While technically this configuration is not a standard Lead II configuration, the trace produced by the electrodes 114A and 114B may be displayed on a standard ECG monitor using the monitor's circuitry to display the trace as a bipolar Lead II trace.

Techniques of using ECG waveforms to locate the tip of a CVC have been available since the 1940's. Some of these prior art devices construct an intravascular ECG trace by placing an electrode near the tip of the CVC and using that electrode to measure the voltage near the tip of the CVC relative to a surface electrode(s) and/or a second electrode spaced from the first.

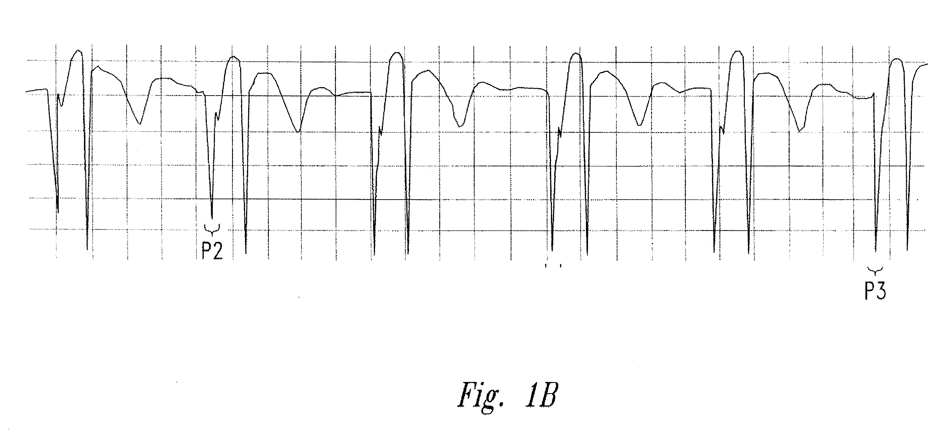

These techniques have shown that both the magnitude and shape of the P wave change depending upon the positioning or location of the electrode attached to the tip of the CVC. Referring to Figures 1A and 1B, two exemplary ECG traces are provided for illustrative purposes.

Figure 1A is an ECG trace made when the electrode attached to the tip of the CVC is in the proximal SVC. This tip location corresponds to position "1" depicted in Figure 2A. The portion of the ECG trace corresponding to an exemplary P wave produced when the electrode attached to the tip is located in position "1" is labeled "P1."

Figure 1B is an ECG trace made when the electrode attached to the tip of the CVC is approaching the SA node and stops at a location adjacent to the SA node. These tip locations correspond to moving the tip from a position "2" to position "3" depicted in Figure 2A. The portion of the ECG trace corresponding to an exemplary P wave produced when the electrode attached to the tip is approaching the SA node is labeled "P2" and the portion of the ECG trace corresponding to an exemplary P wave produced when the electrode attached to the tip is located adjacent to the SA node is labeled "P3."

Normally as the electrode attached to the tip of the CVC moves from the proximal SVC (position "1") toward the SA node (position "3"), the maximum value of the absolute value of the voltage of the P wave increases dramatically. When the electrode attached to the tip of the CVC is adjacent to the SA node (position "3"), the voltage of the P wave (please see "P3" of Figure 1B) reaches a maximum value that is more than twice the value experienced in the proximal SVC and may be as large as eight times the voltage in the proximal SVC. When this occurs, the tip of the CVC is considered to have entered into the right atrium. Because the magnitude of the P wave more than doubles when the electrode attached to the tip of the CVC is adjacent to the SA node, this information may be used to place the tip of the CVC within a few centimeters (e.g., about 1cm to about 2 cm) proximal to the SA node. Additionally, as the electrode attached to the tip of the CVC moves from the proximal SVC toward the right atrium, the shape of the P wave changes from a "u" shape (Figure 1A) to a spike-like shape (Figure 1 B).

Referring to Figure 2B, another exemplary illustration of the P wave portion of the ECG trace produced when the electrode attached to the tip of the CVC is located at positions 1-5 depicted in Figure 2A is provided. The P wave portions of the ECG traces of Figure 2B are labeled with the letter "P" and occur between the vertical dashed lines. Each of the exemplary traces is numbered to correspond to positions "1" through "5." Therefore, the ECG trace "1" was produced when the electrode attached to the tip was located in the proximal SVC. The trace "2" was produced when the electrode attached to the tip was in position "2" (distal SVC). The trace "3" was produced when the electrode attached to the tip was adjacent to the SA node.

As the electrode attached to the tip of the CVC is advanced further into the right atrium, the polarity of the P wave "P" changes from predominantly negative near the top of the right atrium (position "3") to isoelectric (i.e., half has a positive polarity and half has a negative polarity) near the middle of the right atrium (position "4") to almost entirely positive at the bottom of the right atrium (position "5"). These changes in the P wave "P" are illustrated in traces "3" through "5."

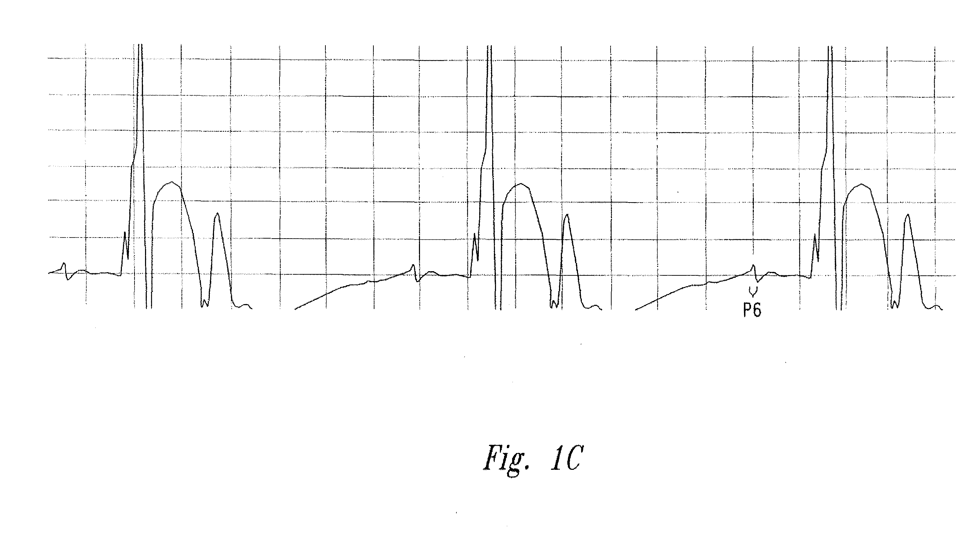

Figure 1C is an ECG trace made when the electrode attached to the tip of the CVC is in the right ventricle. The portion of the ECG trace corresponding to an exemplary P wave produced when the electrode attached to the tip is labeled "P6." When the electrode attached to the tip of the CVC is advanced into the right ventricle, the maximum magnitude of the absolute value of the P wave "P6" approximates the maximum magnitude of the absolute value of the P wave "P1" when the electrode attached to the tip of the CVC was inside the proximal SVC above the SA node (i.e., located at position "1"). However, the polarity of the first half of P wave "P6," which corresponds to the right atrium, is opposite.

The first technique developed for viewing the ECG waveform during the insertion of a CVC used a column of saline disposed within a hollow tube or lumen longitudinally traversing the CVC, The column of saline provides a conductive medium. Saline was inserted into the lumen by a saline filled syringe with a metal needle. The needle of the syringe remained within the entrance to the lumen or port in contact with the column of saline after the lumen was filled. One end of a double-sided alligator clip was attached to the needle and the other end was attached to an ECG lead, which in turn was attached to an ECG monitor. By using the saline solution filled CVC as a unipolar electrode and a second virtual electrode generated by ECG software from three surface electrodes, an intravascular ECG was obtained. The operator would adjust the position of the tip of the CVC based on the magnitude and shape of the P wave displayed by the ECG monitor.

Subsequently, this technique was modified by substituting an Arrow-Johans adapter for the metal needle. The Arrow-Johans adapter is a standard tubing connector with an embedded conductive ECG eyelet. The Arrow-Johans adapter may be placed in line with any conventional CVC. In a closed system, the tubing and CVC may be filled with saline, i.e., a conductive medium, and the CVC used as a unipolar electrode in conjunction with surface electrodes and a standard ECG monitor. The ECG eyelet is placed in contact with the saline in the lumen of the CVC. One end of the ECG lead is attached to the ECG eyelet and the other end to the ECG monitor for displaying the intravascular ECG waveforms. Because the system must be closed to prevent the saline from leaking out, this system works best after the guide wire used to thread the CVC forward has been withdrawn, i.e., after placement has been completed. Therefore, although the catheter may be *withdrawn* after initial placement, it may not be *advanced* into proper position.

BBraun introduced its Certofix catheter to be used in conjunction with its Certodyne adapter. In this system, a patch lead with two ends has an alligator clip connected to one end. The alligator clip is clipped to the CVC guide wire. The other end of the patch lead includes a connector that is plugged into the Certodyne adapter. The ECG may be obtained during placement and the catheter may be advanced or withdrawn as desired. However, the Certodyne adapter has many moving parts and is not sterile, making the procedure cumbersome to perform and the operative field more congested. Additionally, the sterile field may become contaminated by the non-sterile equipment.

The Alphacard, manufactured by BBraun, merges the Arrow-Johans adapter and the Certodyne adapter. The Alphacard consists of a saline filled syringe (used to flush the CVC with saline) and a connector to the Certodyne. The Alphacard is used to obtain a 'snapshot' of the ECG trace from the saline column. If an atrial spike is seen in the ECG trace, the CVC is withdrawn.

With respect to all of these prior art methods of using an ECG trace to place the tip of the CVC, some degree of expertise is required to interpret the P waves measured because the user must advance the guide wire slowly and watch for changes in the P wave. If the catheter is inserted too far too quickly and the changes to the P wave go unnoticed (i.e., the operator fails to notice the increase or spike in the voltage experienced when the electrode attached to the tip is in the right atrium), the operator may mistakenly believe the tip is in the SVC when, in fact, the tip is in the right ventricle, If this occurs, advancing the tip may injure the patient.

United States Patent Nos. 5,078,678 and 5,121,750 both issued to Katims teach a method of using the P wave portion of an ECG trace to guide placement of the tip of the CVC. The CVC includes two empty lumens into which a transmission line is fed or an electrolyte is added. Each of the lumens has a distal exit aperture located near the tip of the CVC. The two exit apertures are spaced from one another. In this manner, two spaced apart electrodes or a single anode/cathode pair are constructed near the tip of the CVC. The voltage or potential of one of the electrodes relative to the other varies depending upon the placement of the electrodes. The voltage of the electrodes is conducted to a catheter monitoring system. The catheter monitoring system detects increases and decreases in the voltage of the P wave. The voltage increases as the electrodes approach the SA node and decrease as the electrodes move away from the SA node. Based on whether the voltage is increasing or decreasing, the operator is instructed by messages on a screen to advance or withdraw the CVC.

While Katims teaches a method of locating the tip of a CVC relative to the SA node, Katims relies on advancing or withdrawing the CVC and observing the changes of the P wave. Katims does not disclose a method of determining the location of the tip of the CVC based on a single stationary position. Unless the entire insertion procedure is monitored carefully, the method cannot determine the position of the tip of the CVC. Further, the Katims method may be unsuitable for determining the location of a previously positioned stationary tip.

Other devices such as Bard's Zucker, Myler, Gorlin, and CVP/Pacing Lumen Electrode Catheters are designed primarily to pace. These devices include a pair of electrodes at their tip that are permanently installed and designed to contact the endocardial lining. These devices include a lumen, which may be used to deliver and/or withdraw medications or fluids as well as for pressure monitoring. These leads are not designed for tip location and do not include multi-lumen capability.

A method of obtaining an intravascular ECG for the purposes of placing a temporary pacing wire was described in United States Patent No. 5,666,958 issued to Rothenberg et al. Rothenberg et al. discloses a bipolar pacing wire having a distal electrode. The distal electrode serves as a unipolar electrode when the pacing wire is inserted into the chambers of the heart. The pacing wire is connected to a bedside monitor through a specialized connector for the purposes of displaying the ECG waveforms detected by the distal electrode.

Given the volume of CVCs placed yearly and the increasing demand particularly for PICC lines (devices that permit the delivery of intravenous therapeutic agents in the outpatient setting, avoiding the need for hospitalization) a great need exists for methods and devices related to locating the tip of a CVC. Particularly, devices and methods are needed that are capable of determining the location of the tip before the operator leaves the bedside of the patient. Further, a method of determining the location (SVC, right atrium, or right ventricle) of the tip from a single data point rather than from a series of data points collected as the catheter is moved may be advantageous. Such a system may be helpful during initial placement and/or repositioning. A need also exists for a device for or a method of interpreting the ECG waveforms that does not require specialized expertise. Methods and devices that avoid the need for hospital and x-ray facilities are also desirable. A need also exists for devices and methods related to determining the position of the tip of the CVC that are less expensive, expose patients to fewer risks, and/or are less cumbersome than the x-ray method currently in use.

### BRIEF DESCRIPTION OF THE SEVERAL VIEWS OF THE DRAWING(S)

Figure 1A is an exemplary ECG trace obtained from an electrode placed inside the proximal SVC.
Figure 1B is an exemplary ECG trace obtained from an electrode approaching the sino-atrial node and stopping adjacent thereto.
Figure 1C is an exemplary ECG trace obtained from an electrode placed inside the right ventricle.
Figure 2A is an illustration of a partial cross-section of the heart providing five exemplary tip locations 1, 2, 3, 4, and 5.
Figure 2B is a series of exemplary P wave traces 1, 2, 3, 4, and 5 obtained from an electrode placed in each of the exemplary tip locations 1, 2, 3, 4, and 5 depicted in Figure 2A, respectively.
Figure 3A is a signal analysis system configured to determine the position of the tip of a CVC using a single pair of electrodes.
Figure 3B is an embodiment of a CVC including three pairs of electrodes.
Figure 4 is a block diagram illustrating a method of using a single pair of electrodes to locate the tip of the CVC within the SVC.
Figure 5 is a block diagram illustrating a method of using a single pair of electrodes to determine the tip of the CVC is located within the right ventricle.
Figure 6 is a block diagram illustrating a method of using a single pair of electrodes to determine the location of the tip of the CVC within the right atrium.
Figure 7 is an embodiment of a signal analysis system for use with the CVC of Figure 3B.
Figure 8 is a block diagram illustrating the components of the signal analysis system of Figure 7.
Figure 9 is block diagram illustrating the components of a monitor of the signal analysis system of Figure 3A.
Figure 10 is a block diagram illustrating a method of using at least two pairs of electrodes to locate the tip of the CVC relative to the SVC, right atrium, and right ventricle.
Figures 11A-11B are a block diagram illustrating an alternate method of using a single pair of electrodes to locate the tip of the CVC relative to the SA node.

### DETAILED DESCRIPTION OF THE INVENTION

### CENTRAL VENOUS CATHETER 100

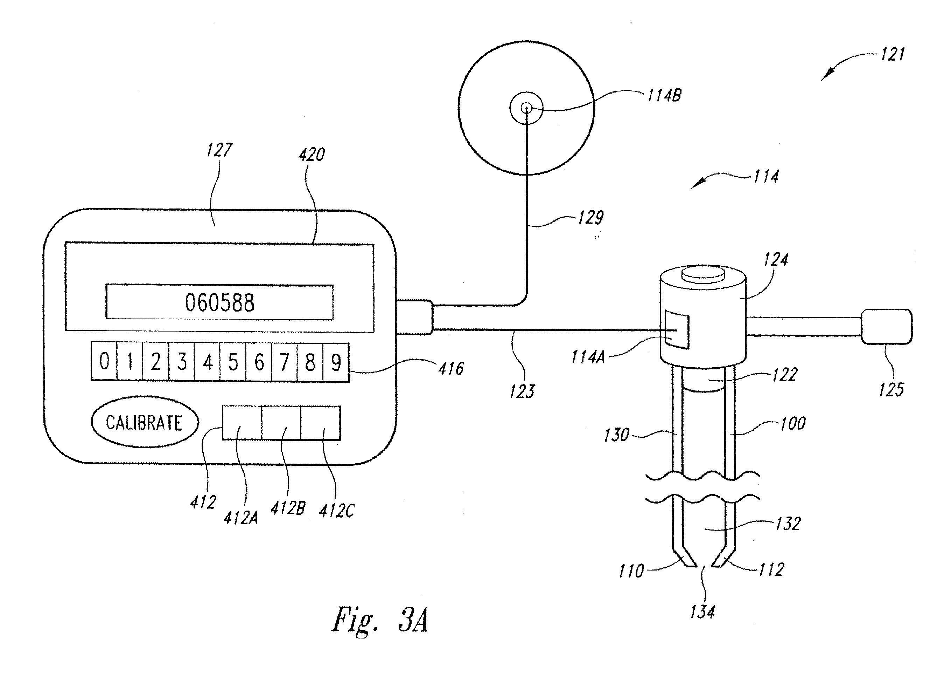

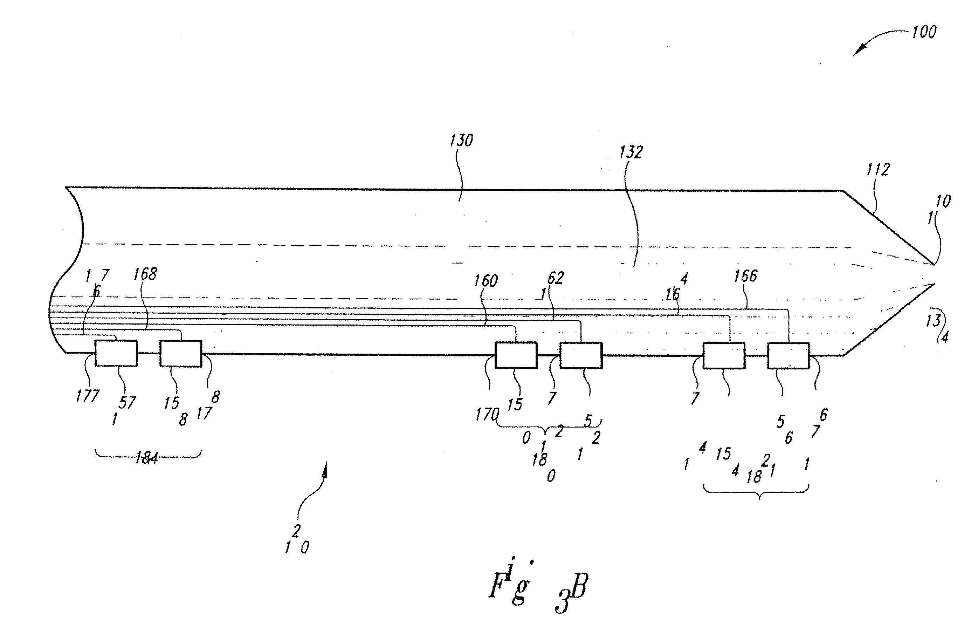

Aspects of the present invention are directed toward a device for locating the tip of a central venous catheter ("CVC") and a method of determining the location of the tip of a CVC. The embodiments depicted in Figures 3A and 3B, include a CVC 100 constructed using any manner known in the art from a flexible nonconductive material, such as polyurethane or other suitable polymer material. It may also be desirable to use a radiopaque material to construct the CVC 100. As is appreciated by those of ordinary skill in the art, the material used to construct the CVC 100 may include materials and/or coatings that provide improved anti-thrombotic or anti-bacterial properties. The CVC 100 has a body 130 configured to be received within a central vein. The body 130 may include a distal end 110 having a tapered tip 112 and a proximal end 120 spaced longitudinally along the body 130 from the distal end 110.

Referring to Figure 3A, the body 130 may include one or more lumens 132 that traverse the length of the body and may have one or more openings 134 at or spaced from the tip 112 and an open end portion 122 configured to permit access into the lumen 132. When the tip 112 of the CVC 100 is received within a central vein, the open end portion 122 may remain outside the central vein allowing materials (e.g., saline, mediations, etc.) to be inserted into the lumen 132 while the tip 112 is inside the central vein or another anatomical structure. The opening 134 permits the passage of materials between the lumen 132 and the environment outside the CVC 100. If one or more materials are inserted into the lumen 132 via the open end portion 122, those materials may exit the lumen 132 via the opening 134. If materials enter the lumen 132 via the opening 134, those materials may exit the lumen 132 via the open end portion 122.

The open end portion 122 is configured be coupled to a connector 124 through which materials may be introduced into or exit from the open end portion 122 of the lumen 132. The connector 124 may include any suitable connector known in the art including a Tuohy-Borst adapter, stop cock, and the like. In the embodiment depicted in Figure 3A, the connector 124 is a Tuohy-Borst adapter, which includes a side port 125 through which materials (e.g., saline) may be introduced into the open end portion 122 of the lumen 132. The side port 125 may be used to flush the lumen 132 with normal saline or another material. The connector 124 is configured to maintain materials within the lumen 132 and to prevent those materials from leaking out of the CVC 100 through the open end portion 122.

The lumens 132 may be used as conduits for the passage of materials such as medications and/or other fluids to and from the environment outside the CVC 100. For example, the lumen 132 may be used to aspirate blood into the CVC 100 and/or provide a conduit through which pressure data may be collected and used to construct pressure waveforms. The environment outside the CVC 100 may include the inside of the SVC, right atrium, and/or right ventricle. The CVC 100 is provided for illustrative purposes and those of ordinary skill in the art appreciate that alternate embodiments of CVC 100 including embodiments with additional lumens, a flow directed balloon tip, thermistors, thermodilution ports, pacing wire ports, embedded pacing electrodes, and the like are within the scope of the present invention.

### DEFLECTION VALUE

The deflection of an ECG trace, (i.e., its vertical height relative to the baseline) may be used to compare two or more P waves. Because a P wave constitutes a voltage change over time, the deflection of the P wave is not constant. In particular embodiments, the P wave is represented by an array or series of discrete numerical values.

The deflection value may be calculated in several ways. For example, the maximum or peak deflection may be used. Alternatively, the deflection value may be calculated as the difference between the maximum deflection and the minimum deflection. The deflection value may also be calculated as the sum of the absolute value of the maximum and minimum deflections. If the P wave has two peaks, which may occur when the tip 112 is located within the right atrium and the P wave is biphasic (see position 4 of Figures 2A and 2B), the deflection value may be calculated by totaling the absolute value of the two peaks. When this method is used, the deflection value of the P wave measured at positions 3-5 may all be approximately equal. Further, if discrete data is being used, the deflection value may also be calculated as a total of the discrete deflection quantities. If continuous data is being used, the deflection value may also be calculated as the integral under the P wave. Further, the deflection value may also be calculated as the average P wave deflection. Because the polarity of portions of the P wave change depending upon the location of the tip 112, it may be beneficial to use the absolute value of the deflection of the P wave to calculate the deflection value.

For the purposes of this application, the term "deflection value" will be used hereafter to describe the metric used to compare two or more P waves, which depending upon the implementation details may be detected by one or more pairs of electrodes. It is appreciated by those of ordinary skill in the art that the deflection value may be determined in numerous ways including those listed above and others not listed and the invention is not limited by the method and manner of determining the deflection value of the P wave.

In the embodiments discussed below, unless otherwise indicated, the deflection value is calculated as the sum of the absolute value of the maximum and minimum deflections when the maximum and minimum deflections have opposite polarities. The deflection value is calculated as the larger of the absolute value of the maximum and minimum deflections when the maximum and minimum deflections have the same polarity. In other words, the vertical height of the P wave is used.

### EMBODIMENTS USING A SINGLE PAIR OF ELECTRODES

Referring to Figure 3A, an embodiment of a system 121 using a single pair of electrodes 114 to determine the position of the tip 112 of the CVC 100 will be described. An electrolytic material or solution, such as saline, may be disposed inside the lumen 132. The electrolytic material inside the lumen 132 forms a continuous conductor or column of electrolytic material that may be used to conduct an electrical signal from the opening 134 in the tip 112, and up the continuous column. In other words, the opening 134 exposes the electrolytic material inside the lumen 132 to electrical activity occurring in the environment outside the tip 112. A first electrode 114A of the pair of electrodes 114 is placed in electrical communication with the continuous column inside the lumen 132. The first electrode 114A may be coupled by a wire 123 to a monitor 127.

A second or surface electrode 114B is placed in contact with the skin of a patient. By way of a non-limiting example, the surface electrode 114B may be affixed to the skin of the patient's chest using any method known in the art. The surface electrode 114B is coupled to the monitor 127 by a wire 129. The voltage at or near the opening 134 in the tip 112 may be measured using the pair of electrodes 114.

The voltages detected by the pair of electrodes 114 may be used to create an ECG trace of the electrical activity observed at or near the tip 112 of the CVC 100. Because the voltage across each of the pair of electrodes 114 may vary with time, the voltage across wires 123 and 129 may constitute a time-varying signal that can be analyzed using standard signal processing methods well known in the art. In a typical patient, the maximum voltage across the pair of electrodes 114 may range from about 0.2 mV to about 3 mV. The signal detected by the pair of electrodes 114 may be amplified and/or filtered to improve the signal quality.

In the embodiment depicted in Figure 3A, the first electrode 114A is coupled to the connector 124. Alternatively, the first electrode 114A may be located inside at least a portion of the lumen 132. By way of another example, the first electrode 114A is coupled to the side port 125 through which the electrolytic material (e.g., saline) may be introduced into the open end portion 122 of the lumen 132. As is apparent to those of ordinary skill in the art, the first electrode 114A may be located anywhere that would place it in electrical continuity or communication with the electrolytic material (e.g., saline) exiting the tip 112 via the opening 134 or otherwise communicating electrically with the environment outside the opening 134 of the tip 112. By way of another example, the first electrode 114A may be incorporated into a guide wire (not shown), stylet, and the like that extends from, or near the tip 112 up the body 130 of the CVC 100 and is electrically coupled by the wire 123 to the monitor 127. The monitor 127 is described in detail below.

### METHOD OF USING A SINGLE ELECTRODE PAIR TO DETERMINE THE POSITION OF THE TIP OF THE CVC

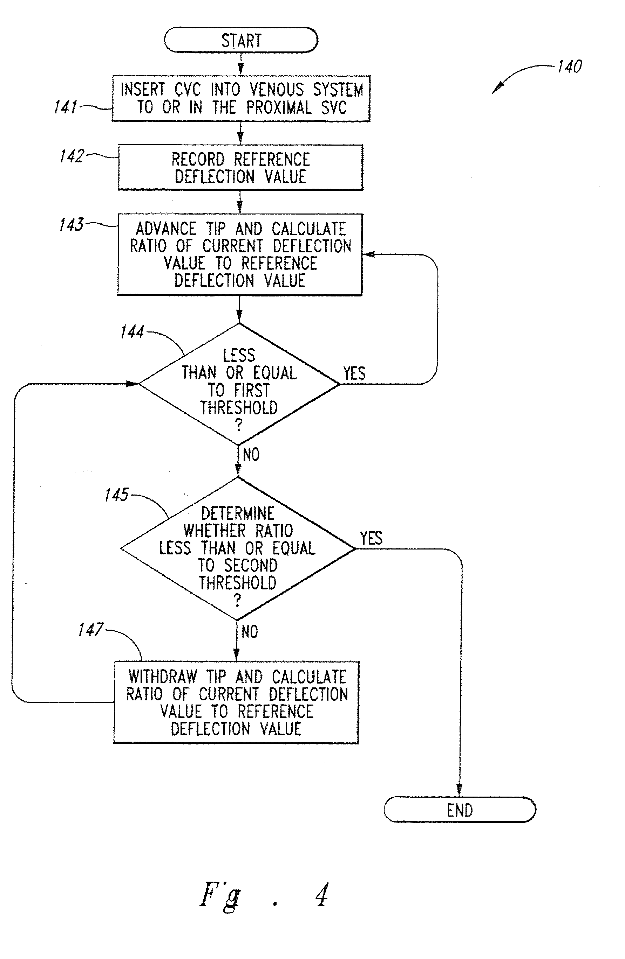

Referring to Figure 4, a method 140 of determining the location of the tip 112 using the pair of electrodes 114 will now be described. In block 141, the CVC 100 is inserted into the SVC. The CVC 100 may gain venous access to the SVC by any method known in the art including inserting the CVC 100 in a standard sterile fashion through the subclavian, one of the jugular veins, or a peripheral vein and directing the tip 112 of the CVC 100 through that vein toward the proximal SVC.

Next, in block 142, a reference deflection value is recorded in a storage location. The reference deflection value is the deflection value obtained from the pair of electrodes 114 when the tip 112 is located in the venous system proximal to or in the proximal SVC.

Then, in block 143, the tip 112 is advanced. As the tip 112 is advanced, a ratio of the deflection value of the currently observed P wave to the reference deflection value is calculated. Inside the SVC, as the tip 112 approaches the mid SVC, the deflection value of the P wave may increase by two to four times the reference deflection value. Further, as the tip 112 approaches the distal SVC, the deflection value of the P wave may increase by four to six times the reference deflection value. In the distal SVC near the SA node, the deflection value of the P wave may increase by six to eight times the reference deflection value.

In decision block 144, whether the ratio is less than or equal to a first predefined threshold value is determined. The first predefined threshold value should be large enough to ensure the tip 112 has left the proximal SVC and entered the mid SVC. Further, the first predefined threshold value should be small enough to prevent placement of the tip 112 in the distal SVC. Thus, the first predefined threshold value may be within a range of about 1.6 to about 3.9. By way of a non-limiting example, the first predefined threshold value may be about 2.0. If the ratio is less than or equal to the first predefined threshold value, in block 143, the tip 112 may be advanced and the ratio recalculated. Ratio values less than or equal to the first predefined threshold value indicate the tip 112 is in the proximal or mid SVC and has not yet reached either the distal SVC, caval-atrial junction adjacent to the SA node or the upper right atrium.

If decision block 144 determines the ratio is not less than or equal to the first predefined threshold value, the method 140 advances to decision block 145. In decision block 145, whether the ratio is less than or equal to a second predefined threshold value is determined. The second predefined threshold value should be large enough to ensure the tip 112 is in the distal SVC and small enough to avoid entry of the tip 112 into the right atrium. By way of a non-limiting example, the second predefined threshold value may within a range of about 4.0 to about 8.0. By way of a non-limiting example, the second predefined threshold value may be about 8.0. In other words, the block 144 determines whether the ratio is between the first and second threshold values or within a range defined between the first and second threshold values (e.g., about 2.0 to about 8.0). Nevertheless, ratio values within the range defined between the first and second threshold values may indicate the tip 112 is approaching or has reached the SA node, or the tip is located within the right atrium. In other words, any ratio value above the second predefined threshold value may indicate the tip 112 is located in the distal SVC or the upper atrium. If the ratio is within the range defined between the first and second threshold values, the decision in decision block 145 is "YES" and the method 140 ends.

If the ratio is greater than the second threshold value, the decision in decision block 145 is "NO," and the method 140 advances to block 147. When the ratio is greater than the second threshold value, the tip 112 is in or near the right atrium and in block 147, the user or operator is advised to withdraw the tip 112. By way of a non-limiting example, the user or operator may be advised to withdraw the tip 112 about 0.5 cm to about 1 cm. Then, in block 147, advancement of the tip 112 is terminated and the tip 112 withdrawn. By way of a non-limiting example, the user or operator may withdraw the tip 112 about 0.5 cm to about 1 cm. In block 147, as the tip 112 is withdrawn, the ratio of the deflection value of the currently observed P wave to the reference deflection value is recalculated.

Then, the method 140 returns to decision block 144.

In particular embodiments, if at any time during the performance of method 140, the ratio is equal to the second predefined threshold value, the tip 112 is maintained in its current position without additional advancement or withdrawal and the method 140 ends. When the method 140 ends, the ratio is between the first and second threshold values and the tip 112 is located in the mid SVC or distal SVC.

The following table, Table 1, summarizes the relationship between the location of the tip 112 of the CVC 100 and the ratio of the deflection value of the currently observed P wave to the reference deflection value:

**Table 1**

| Location of the tip 112 | Proximal SVC | Mid SVC | Distal SVC | Very Distal SVC or Right Atrium |
|---|---|---|---|---|
| **Ratio:** ratio of the deflection value of the currently observed P wave to the reference deflection value | ≤1.5 | 1.6-4.0 | 4.1-5.5 | >5.5-8.0 |

While Table 1 provides exemplary ranges and/or threshold values for use as a general guideline, those of ordinary skill in the art appreciate that these values may benefit from adjustment as additional anatomic or electrophysiologic data is acquired and such modified values are within the scope of the present invention.

As is apparent to those of ordinary skill, the pair of electrodes 114 may be used to detect the instantaneous location of the tip 112. Therefore, if the tip 112 migrates into the atrium, this movement may be detected immediately by calculating a ratio of the deflection value of the currently observed P wave to the recorded reference deflection value and comparing the ratio to the first and second threshold values. This calculation may be performed periodically or at random intervals. If the tip 112 migrates into the atrium, a medical professional may be alerted via a signal, such as an alarm, and the like, to reposition the tip 112.

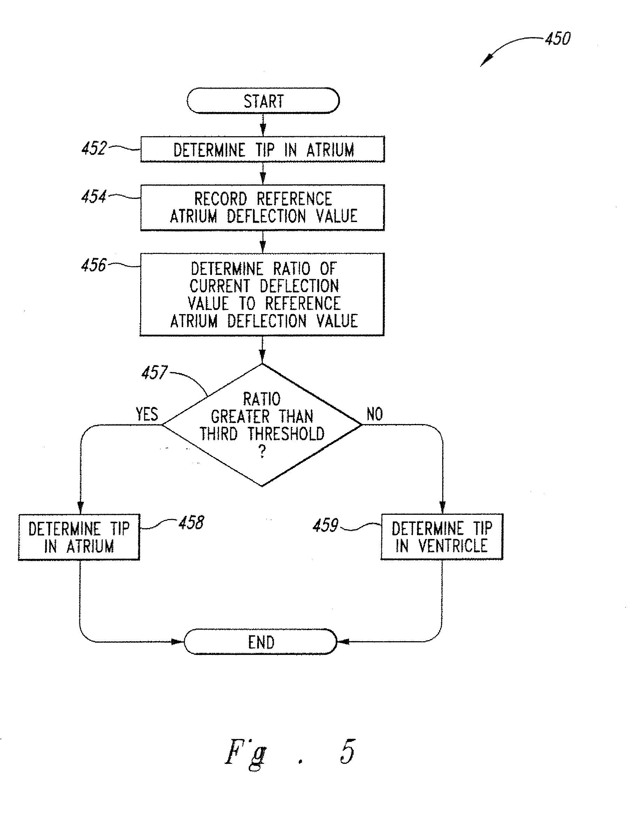

Referring to Figure 5, a method 450 may be used to determine the tip 112 is located within the ventricle. In first block 452, the tip 112 is located in the atrium. Then, in block 454, a reference atrium deflection value is recorded. The reference atrium deflection value is the deflection value obtained from the pair of electrodes 114 when the tip 112 is located in the atrium. Then, in block 456, a ratio of the deflection value of the current P wave detected by the pair of electrodes 114 to the reference atrium deflection value is determined. In decision block 457, whether the ratio is greater than a third predefined threshold value is determined. The third predefined threshold value may be about 0.5. If the decision is "YES," the ratio is greater than the third predefined threshold value, and in block 458, the tip 112 is determined to be in the right atrium. Then, the method 450 ends. If the decision in decision block 457 is "NO," the ratio is less than or equal to the third predefined threshold value, and in block 459, the tip 112 is determined to be in the right ventricle. Then, the method 450 ends.

Within at least a portion of the range defined between the first and second threshold values (e.g., about 2.0 to about 8.0), the P wave voltage measured using prior art techniques, such as those disclosed by United States Patent Nos. 5,078,678 and 5,121,750 (both issued to Katims), has generally not yet reached its maximum value. Therefore, the present method indicates the tip 112 should be withdrawn before those techniques would signal withdrawal. For example, typically the P wave voltage within the proximal SVC is about 0.2 to about 0.3 mV. Near the SA node, the P wave voltage may increase about 8 fold (e.g., about 1.6 mV to about 2.4 mV. In other words, the ratio is about 8 near the SA node, which is the location of the maximum P wave voltage used in the prior art. However, prior art techniques advise to advance the tip until it can be clearly seen that the maximum P voltage has been reached. Therefore, the prior art techniques allow the tip 112 to advance further into the right atrium than the present technique before identifying the advancement should be halted and the tip withdrawn back into the SVC. Because the present technique halts the advancement of the tip 112 earlier (e.g., when the P wave voltage increases above the second predefined threshold value of about 8.0, which corresponds to about 1.6 mV to about 2.4 mV) than prior art techniques, the present teachings may avoid many of the risks associated with advancing the tip into the right atrium.

Further, the prior art teaches using a threshold percentage decrease to determine the tip 112 is in the correct location. However, using a threshold percentage decrease may be ineffective at locating the tip 112 within the SVC because the percentage decrease may vary from patient to patient. In other words, depending upon the anatomical structures of the patient, the tip 112 may have to be withdrawn until the percentage decreases differing amounts. On the other hand, if the tip 112 is withdrawn until the ratio is between about 2.0 and about 8.0 (e.g., the current P wave voltage is about 0.4 mV to about 2.4 mV), the tip will be located in the mid SVC or in some cases, the distal SVC. Therefore, the present technique more accurately positions the tip 112 than prior art techniques.

In addition to halting the advancement of the tip 112 when the ratio of the deflection value of the currently observed P wave to the reference deflection value has exceeded either of the first and second predefined threshold values, advancement of the tip 112 could also be halted when the deflection value of the currently observed P wave is approximately equivalent to the voltage (or deflection value) of the QRS complex.

As discussed above, when observed using a standard bipolar Lead II trace, the P wave voltage is almost entirely negative at the top of the right atrium (see trace 3 of Figure 2B), biphasic in the mid right atrium (see trace 4 of Figure 2B), and positive at the bottom of the right atrium (see trace 5 of Figure 2B). A positive/total deflection ratio may be calculated and used to determine when advancement of the tip 112 should be halted. The positive/total deflection ratio is a ratio of the greatest positive deflection value (of an initial positive or upwardly deflecting portion that precedes a downwardly deflecting portion) to the total deflection value of the currently observed P wave. When the positive/total deflection ratio is greater than a predetermined fraction (e.g., one quarter, one eighth, etc.) advancement of the tip 112 should be halted. For example, the traces 3-5 illustrated in Figure 2B each have an initial upwardly deflecting portion. However, in both cases, the greatest positive deflection value of the initial upwardly deflecting portion of the P wave is clearly greater than one quarter of the total deflection value of the currently observed P wave. As explained above, in these traces, the tip 112 is located in the right atrium. Thus, if the greatest positive deflection value is greater than the predetermined fraction of the total deflection value of the currently observed P wave, the tip 112 is in the right atrium and should be with drawn.

### ALTERNATE METHOD OF USING A SINGLE ELECTRODE PAIR TO DETERMINE THE POSITION OF THE TIP OF THE CVC

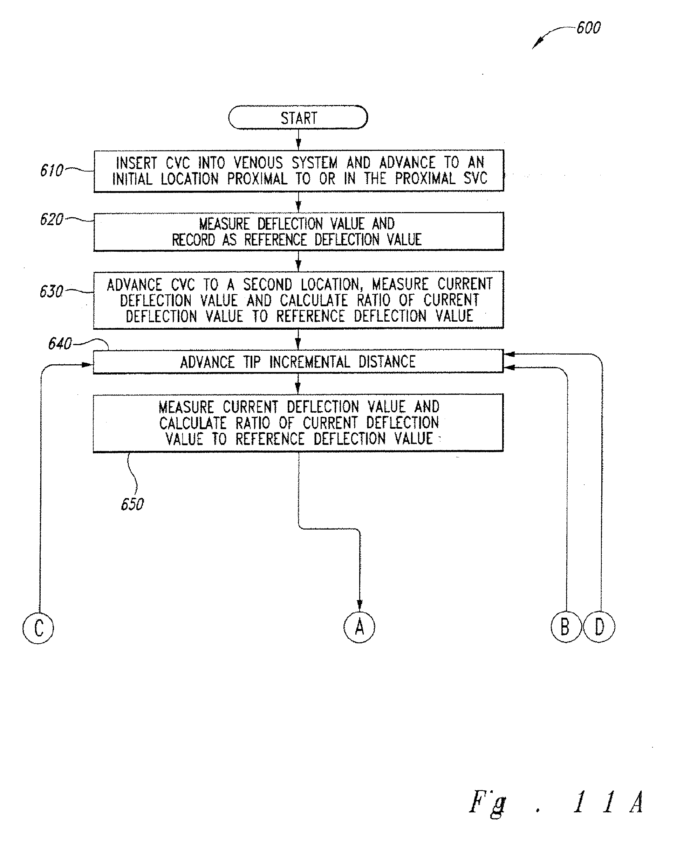

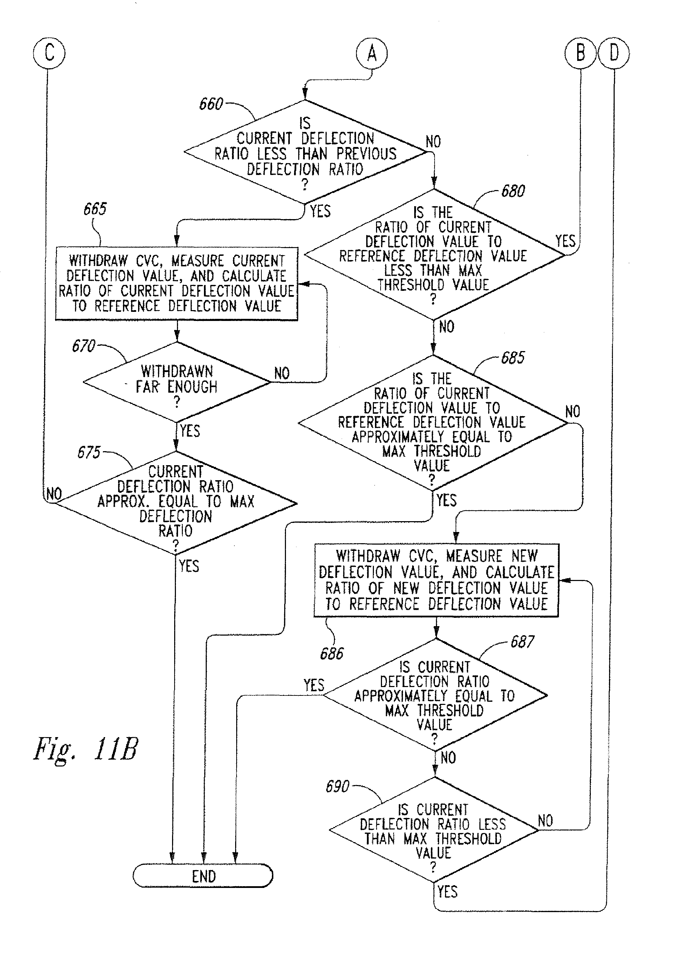

Figures 11A and 11B provide a block diagram of an alternate method 600 of advancing and locating the tip 112 of the CVC 100. A physical cathode-anode electrode pair is used in a standard bipolar lead setup. A bipolar lead setup means two physical leads are used (rather than one virtual lead and one physical lead, which are referred to as a unipolar lead setup).

For illustrative purposes, the method 600 is described using the first electrode 114A (see Figure 3A) functions as a cathode at the tip 112 and the second electrode 114B (see Figure 3A) functions as an anode attached to the patient's skin near his/her right shoulder. The continuous conductor or column inside the lumen 132, which is in electrical communication with both the first electrode 114A and the electrical activity occurring in the environment outside the tip 112 functions as a "wandering electrode," which is the positive cathode. The second electrode 114B serves as the negative anode electrode. While technically this configuration is not a standard Lead II configuration, the trace produced by the electrodes 114A and 114B may be displayed on a standard ECG monitor using the monitor's circuitry to display the trace as a bipolar Lead II trace. Thus, an ECG trace is generated for the wandering electrode relative to the electrode "RA," which is displayed as a Lead II trace. However, as is apparent to those of ordinary skill in the art, through application of ordinary skill in the art to the present teachings other ECG Lead traces could be used and are within the scope of the present disclosure.

As is apparent to those of ordinary skill in the art, in a standard unipolar lead setup, three surface electrodes (not shown) are attached to a patient's skin: an electrode "RA" is attached to the patient's right arm (or shoulder), an electrode "LA" is attached to the patient's left arm (or shoulder), and an electrode "LL" is attached to the patient's left leg. A virtual electrode may be created using ECG software, which calculates the virtual electrode as the electrical center of an Einthoven's triangle created by the electrodes "RA," "LA," and "LL" used in the Lead I, Lead II, and Lead III configurations. The continuous conductor or column inside the lumen 132, which is in electrical communication with both the first electrode 114A and the electrical activity occurring in the environment outside the tip 112 functions as a "wandering electrode." The wandering electrode is the positive cathode, and the virtual electrode is the negative anode electrode. Thus, an ECG trace is derived for the wandering electrode relative to the virtual electrode. In addition to Leads I, II, and III (of the bipolar configurations), some ECG monitors display unipolar lead configurations, e.g., aVR, aVL, aVF derived from a composite of the electrodes "RA," "LA" and "LL," or a chest electrode, variably called a "V," "MCL," or "Chest" lead (hereafter referred to as the "V" lead). Some ECG monitors display this ECG trace as a unipolar "V" lead trace and some users particularly like to use the unipolar "V" lead trace to guide the tip 112 of the CVC 100. However, most users use the bipolar Lead II trace generated for the wandering electrode relative to the electrode "RA" (i.e., the ECG Lead II configuration discussed above). As is apparent to those of ordinary skill in the art, with respect to the method 600, any number of bipolar or unipolar lead configurations may be used with acceptable results. Further, either the unipolar "V" lead trace or the bipolar Lead II trace (among others) may be used to perform the method 140.

In method 600, the deflection values measured include only the negative polarity or downwardly extending portion of the P-wave. The upwardly extending positive polarity portion is not included in the measurement of the deflection values. Thus, the deflection value is calculated as the absolute value of the minimum deflection.

In first block 610, the CVC 100 is introduced into the venous system and advanced to an initial location estimated to place the tip 112 of the CVC 100 at or proximal to the proximal SVC. In next block 620, a deflection value of the P wave observed at the initial location is measured and stored as a reference deflection value.

Then, in block 630, the CVC 100 is advanced from the initial location to a second location. By way of a non-limiting example, the CVC 100 may be advanced from the initial location about 0.5 cm to about 1.0 cm in block 630. At the second location, a new deflection value is measured using of the P wave observed at the second location. A deflection ratio of the new deflection value to the reference deflection value is then calculated. As the method 600 is performed, a maximum deflection ratio observed is stored. Thus, in block 630, the maximum deflection value observed is equal to the deflection ratio observed at the second location.

In block 640, the CVC 100 is advanced from the second location by an incremental distance. By way of a non-limiting example, the incremental distance may be within a range of about 0.5 cm to about 1.0 cm. However, a smaller sized incremental distance may be used and is within the scope of the present teachings.

After the CVC 100 has been advanced by the incremental distance, in block 650, a current deflection value of the P wave observed at the new location is measured and a current deflection ratio of the current deflection value to the reference deflection value is calculated. If the current deflection ratio is greater than the maximum deflection ratio, the maximum deflection ratio is set equal to the current deflection ratio. Before the CVC 100 was advanced, the CVC was located a previous location. The deflection value measured at the previous location is a previous deflection value and the deflection ratio of the previous deflection value to the reference deflection value is a previous deflection ratio.

Then, in decision block 660, the current deflection ratio is compared to the previous deflection ratio. The decision in decision block 660 is "YES" when the current deflection ratio is less than the previous deflection ratio. Otherwise, the decision in decision block 660 is "NO."

When the decision in decision block 660 is "YES," in block 665, the CVC 100 is withdrawn from the current location to a new location. By way of a non-limiting example, in block 665, the CVC 100 may be withdrawn about 0.5 cm to about 1.0 cm. Then, a new deflection value of the P wave observed at the new location is measured and a new deflection ratio of the new deflection value to the reference deflection value is calculated. If the new deflection ratio is greater than the maximum deflection ratio, the maximum deflection ratio is set equal to the new deflection ratio. At this point, the location of the CVC 100 before withdrawal is a previous location and the deflection ratio calculated at the previous location is a previous deflection ratio. The location of the CVC 100 after withdrawal is a current location and the deflection ratio calculated at the current location is a current deflection ratio. By way of a non-limiting example, the CVC 100 may be withdrawn about 1 cm. Then, the method 600 advances to decision block 670.

In decision block 670 whether the CVC 100 has been withdrawn far enough is determined. In decision block 670, a positive/total deflection ratio of the greatest positive deflection value (of an initial positive or upwardly deflecting portion which precedes a downwardly deflecting portion) to the total deflection value of the currently observed P wave is calculated. The decision in decision block 670 is "YES" when the positive/total deflection ratio is less than a predetermined fraction (e.g., one quarter, one eighth, etc.). Otherwise, the decision in decision block 670 is "NO."

Alternatively, in decision block 670, a negative/total deflection ratio of the smallest negative deflection value (of a negative polarity or downwardly deflecting portion of the currently observed P wave which follows an upwardly deflecting positive polarity portion of the currently observed P wave) to the total deflection value of the currently observed P wave is calculated. The decision in decision block 670 is "YES" when the negative/total deflection ratio is greater than or equal to a predetermined fraction (e.g., three quarters, seven eighths, etc.). Otherwise, the decision in decision block 670 is "NO."

When the decision in decision block 670 is "YES," the method 600 advances to decision block 675. When the decision in decision block 670 is "NO," the method 600 returns to block 665.

In decision block 675, the current deflection ratio is compared to the maximum deflection ratio observed. The decision in decision block 675 is "YES" when the current deflection ratio is approximately equal to the maximum deflection ratio observed. By way of a non-limiting example, the current deflection ratio may be considered approximately equal to the maximum deflection ratio observed when the absolute value of the difference between the current deflection ratio and the maximum deflection ratio observed is less than 0.2. If the current deflection ratio is not approximately equal to the maximum deflection ratio observed, the decision in decision block 675 is "NO."

When the decision in decision block 675 is "YES," the method 600 ends. When the decision in decision block 675 is "NO," the method 600 returns to block 640.

When the decision in decision block 660 is "NO," the current deflection ratio (calculated after the CVC 100 was advanced by the current incremental distance) is greater than or equal to the previous deflection ratio (calculated after the CVC 100 was advanced by the previous incremental distance). When the decision in decision block 660 is "NO," the method 600 advances to decision block 680.

The decision in decision block 680 is "YES," when the current deflection ratio is less than a maximum threshold value. Otherwise, decision in decision block 680 is "NO." By way of a non-limiting example, the maximum threshold value may be about 8.0.

When the decision in decision block 680 is "YES," the current deflection ratio is less than the maximum threshold value (e.g., 8.0) and greater than the previous deflection ratio. When this is the case, the method 600 returns to block 640.

When the decision in decision block 680 is "NO," the current deflection ratio is greater than or equal to the maximum threshold value (e.g., 8.0), and the method 600 advances to decision block 685 to determine whether the current deflection ratio is approximately equal to the maximum threshold value.

When the current deflection ratio is approximately equal to the maximum threshold value, the decision in decision block 685 is "YES." Otherwise, decision in decision block 685 is "NO." The current deflection ratio may be considered approximately equal to the maximum threshold value when the absolute value of the difference between the current deflection ratio and the maximum threshold value is less than 0.2.

When the decision in decision block 685 is "YES," the method 600 ends. When the decision in decision block 685 is "NO," the current deflection value is neither less than nor equal to the maximum threshold value. When this occurs, the method 600 progresses to block 686 whereat the CVC 100 is withdrawn from the current location to a new location. Then, a new deflection value of the P wave observed at the new location is measured and a new deflection ratio of the new deflection value to the reference deflection value is calculated. The location of the CVC 100 after withdrawal is a current location and the deflection ratio calculated at the current location is a current deflection ratio. By way of a non-limiting example, the CVC 100 may be withdrawn about 1 cm. Then, the method 600 advances to decision block 687.

In decision block 687, the current deflection ratio is compared to the maximum threshold value. The decision in decision block 670 is "YES" when the current deflection ratio is approximately equal to the maximum threshold value. By way of a non-limiting example, the current deflection ratio may be considered approximately equal to the maximum threshold value when the absolute value of the difference between the current deflection ratio and the previous deflection ratio is less than 0.2. When the current deflection ratio is not approximately equal to the maximum threshold value, the decision in decision block 687 is "NO."

When the decision in decision block 687 is "YES," the method 600 ends. When the decision in decision block 670 is "NO," the method 600 advances to decision block 690. Decision block 690 determines whether the CVC 100 was withdrawn too far in block 686. The decision in decision block 690 is "YES" when the current deflection ratio is less than maximum threshold value, When this occurs, continuing to withdraw the CVC 100 will simply reduce the current deflection value. Thus, to make the current deflection ratio approximately equal to the maximum threshold value, the CVC 100 must be advanced.

The decision in decision block 690 is "NO" when the current deflection ratio is greater than the maximum threshold value. Thus, to make the current deflection ratio approximately equal to the maximum threshold value, the CVC 100 must be withdrawn. When the decision in decision block 690 is "NO," the method 600 returns to block 686.

If at anytime during the performance of the method 600, the current deflection ratio is approximately equal to the previously observed maximum deflection ratio, advancement and withdrawal of the CVC 100 may be halted and performance of the method 600 terminated. Similarly, if at anytime during the performance of the method 600, the current deflection ratio is approximately equal to the maximum threshold value, advancement and withdrawal of the CVC 100 is halted and performance of the method 600 terminated.

### METHOD OF USING A SINGLE ELECTRODE PAIR TO DETERMINE THE LOCATION OF THE TIP OF THE CVC WITHIN THE RIGHT ATRIUM

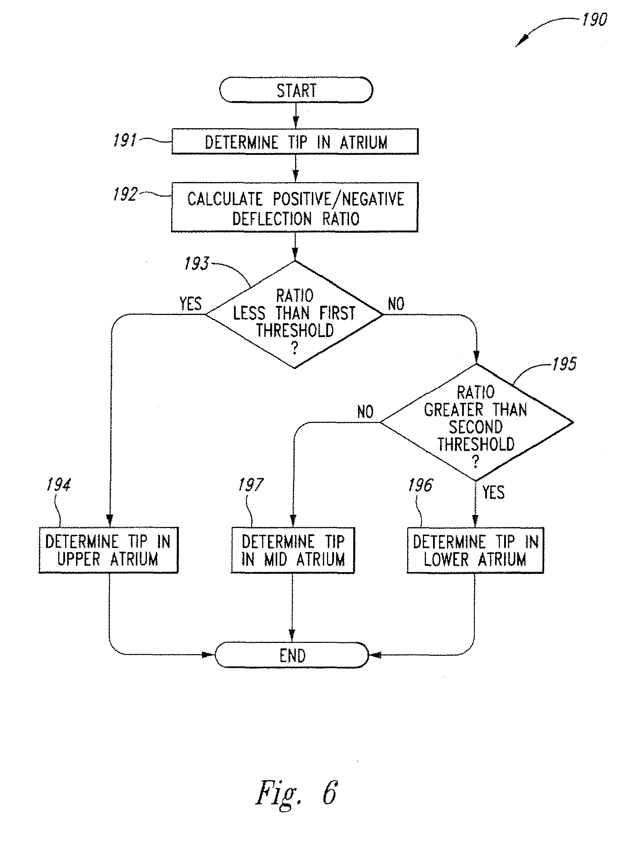

As discussed above, the P wave voltage is almost entirely negative at the top of the right atrium (see trace 3 of Figure 2B), biphasic in the mid right atrium (see trace 4 of Figure 2B), and positive at the bottom of the right atrium (see trace 5 of Figure 2B). Referring to Figure 6, a method 190 uses these characteristics of the P wave voltage to determine the location of the tip 112 of the CVC 100 within the atrium. As is apparent to those of ordinary skill in the art, with respect to the method 190, either the unipolar "V" lead trace or the bipolar Lead II trace may be used.

In block 191, any method known in the art or described herein is used to determine the tip 112 is located in the atrium. For example, the tip 112 is in the atrium when the ratio of the deflection value of the currently observed P wave to the reference deflection value is greater than a value that may vary from person to person but is within a range of about 4.0 to about 8.0. Alternatively, the tip 112 may be determined to be in the atrium when the P wave voltage has exceeded a predetermined amount (e.g., about 0.8 mV to about 2.4 mV). Further, the tip 112 may be determined to be in the atrium when the positive/total deflection ratio (i.e., a ratio of the greatest positive deflection value of the initial upwardly deflecting portion of the currently observed P wave, which precedes a downwardly deflecting portion, to the total deflection value) is greater than the predetermined fraction (e.g., one quarter, one eighth, etc.). By way of another example, the tip 112 may be determined to be in the atrium when the voltage (or deflection value) of the currently observed P wave is approximately equivalent to or greater than the voltage (or deflection value) of the QRS complex.

After it is determined the tip 112 is in the right atrium, in block 192, a positive/negative deflection ratio is calculated. The positive/negative deflection ratio is a ratio of the greatest positive deflection value to the smallest negative deflection value. As discussed above, the absolute value of the deflection values may used. Thus, the positive/negative deflection ratio may be calculated as a ratio of the deflection value having the largest absolute value within the portion of the P wave that has a positive polarity to the deflection value having the largest absolute value within the portion of the P wave having a negative polarity. If the P wave is entirely negative, the positive/negative ratio is zero (and the tip 112 is in the upper atrium). On the other hand, if the largest positive deflection value and the smallest negative deflection value are equal, the positive/negative deflection ratio is equal to one. In subsequent blocks 193-197, the positive/negative deflection ratio is used to determine whether the tip 112 is located in the upper, mid, or lower atrium.

In decision block 193, whether the positive/negative deflection ratio is less than a first predetermined threshold value is determined. The first predetermined threshold value may be about 0.80. If decision block 193 determines the ratio is less than the first predetermined threshold value, in block 194, the method 190 determines the tip 112 is in the upper atrium and the method 190 ends.

If decision block 193 determines the ratio is not less than the first predetermined threshold value, the method 190 advances to decision block 195. In decision block 195, whether the positive/negative deflection ratio is greater than a second predetermined threshold value is determined. The second predetermined threshold value may be about 1.20. If decision block 195 determines the ratio is greater than the second predetermined threshold value, in block 196, the method 190 determines the tip 112 is in the lower atrium and the method 190 ends.

If decision block 195 determines the ratio is not greater than the second predetermined threshold value, in block 197, the method 190 determines the tip 112 is in the mid atrium and the method 190 ends. In other words, if the positive/negative deflection ratio is between the first and second predetermined threshold values, the tip 112 is in the mid atrium. Further, if the positive/negative deflection ratio is equal to the first predetermined threshold value or the second predetermined threshold value, the tip 112 is in the mid atrium.

The following table summarizes the relationship between the location of the tip 112 of the CVC 100 and the positive/negative deflection ratio:

**Table 2**

| Location of the tip 112 | Upper Atrium | Mid Atrium | Lower Atrium |
|---|---|---|---|
| **Positive/Negative Deflection Ratio:** ratio of the greatest positive deflection value to the smallest negative deflection value | < 0.80 | 0.80-1.20 | > 1.20 |

While Table 2 provides exemplary ranges and/or threshold values for use as a general guideline, those of ordinary skill in the art appreciate that these values may benefit from adjustment as additional anatomic or electrophysiologic data is acquired and such modified values are within the scope of the present invention.

### EMBODIMENTS USING TWO OR MORE PAIRS OF ELECTRODES

In the embodiment depicted in Figure 3B, the CVC 100 includes four longitudinally spaced apart electrodes 150, 152, 154, and 156. Each electrode 150, 152, 154, and 156 is in electrical communication with a wire 160, 162, 164, and 166, respectively. In particular embodiments, the electrodes 150, 152, 154, and 156 are constructed from the distal end of each of the wires 160, 162, 164, and 166. In another embodiment, the electrodes 150, 152, 154, and 156 are attached to the ends of the wires 160, 162, 164, and 166 by any method known in the art for attaching an electrode to a wire, including soldering. The wires 160, 162, 164, and 166 are electrically isolated from one another. The wires 160, 162, 164, and 166 may be insulated from the environment outside the body 130 by the body 130.

The electrodes 150, 152, 154, and 156 and the wires 160, 162, 164, and 166 may be constructed from any suitable materials known in the art such as stainless steel or platinum. Alternatively, a column of conductive material such as an electrolytic material (e.g., saline) may be used to construct one or more of the electrodes 150, 152, 154, and 156 and/or the wires 160, 162, 164, and 166. The electrodes 150, 152, 154, and 156 may be about 6 mm to about 12 mm long, about 6 mm to about 12 mm wide, and about 1 mm to about 4 mm thick. The wires 160, 162, 164, and 166 may be constructed using any electrical lead wire suitable for obtaining an ECG trace.

Optionally, the invention may include two longitudinally spaced apart electrodes 157 and 158. Each of the electrodes 157 and 158 may be electrical communication with a wire 167 and 168, respectively. The electrodes 157 and 158 and wires 167 and 168 may be constructed in a manner substantially similar to that used to construct the electrodes 150, 152, 154, and 156 and the wires 160, 162, 164, and 166, respectively. In particular embodiments, the electrode 157 and 158 are positioned proximal to the electrodes 150, 152, 154, and 156.

Electrodes 150, 152, 154, and 156 may form two anode/cathode pairs. For example, electrodes 150 and 152 may form a first or proximal anode/cathode pair 180 and electrodes 154 and 156 may form a second or distal anode/cathode pair 182. Optional electrodes 157 and 158 may form an optional third or reference anode/cathode pair 184. A pair of electrodes forming an anode/cathode pair may be attached to a pair of insulated wires housed within a single cable. In particular embodiments, a pair of bipolar lead wires are used. In this manner, the four electrodes of the proximal and distal anode/cathode pairs 180 and 182 may be attached to two lead wires. A third bipolar lead wire may be included for use with the reference anode/cathode pair 184. Alternatively, the proximal and distal anode/cathode pairs 180 and 182 may be attached to four insulated wires housed within a single cable such a dual bipolar lead wire.

The wires 160, 162, 164, and 166 and electrodes 150, 152, 154, and 156 may be permanently embedded into the body 130 of the CVC 100 or removably inserted into one or more channels or lumens 132 formed in the CVC 100 for potential future removal and/or replacement. The wires 167 and 168 and electrodes 157 and 158 may be incorporated into the CVC 100 in any manner described with respect to wires 160, 162, 164, and 166 and electrodes 150, 152, 154, and 156, respectively.

The electrodes 150, 152, 154, and 156 are in electrical communication with the environment outside the CVC 100. In particular embodiments, a portion of each of the electrodes 150, 152, 154, and 156 are exposed to the environment outside the CVC 100 by apertures 170, 172, 174, and 176 formed in the body 130 adjacent to the electrodes 150, 152, 154, and 156, respectively. In embodiments including optional electrodes 157 and 158, a portion of each of the electrodes 157 and 158 may be exposed to the environment outside the CVC 100 by apertures 177 and 178 formed in the body 130 adjacent to the electrodes 157 and 158, respectively. The apertures 177 and 178 may be constructed in any manner suitable for constructing apertures 170, 172, 174, and 176. The apertures 170, 172, 174, and 176 may be formed in the body 130 by any method known in the art and the invention is not limited by the method used to construct the apertures 170, 172, 174, and 176. While the electrodes 150, 152, 154, and 156 depicted in the drawings extend outwardly from the body 130 through the apertures 170, 172, 174, and 176, it is understood by those of ordinary skill in the art, that electrodes 150, 152, 154, and 156 may reside at the bottom of the apertures 170, 172, 174, and 176 which may provide a passageway for fluids in the outside environment to the electrodes 150, 152, 154, and 156. Alternatively, the portion of the electrodes 150, 152, 154, and 156 in electrical communication with the environment outside the CVC 100 may be flush with the outside surface of the CVC 100.

The electrode 156 may be located at or spaced from the tip 112. In particular embodiments, the electrode 156 is less than about 5 mm from the tip 112. The spacing between an anode and cathode of the anode/cathode pairs 180 and 182 may be about 1 mm to about 4 mm. In particular embodiments, the spacing between an anode and cathode of the anode/cathode pairs 180 and 182 is about 3 mm.

In particular embodiments, the distance between the electrodes 154 and 152 is less than the height of the right atrium. In an adult, the height of the right atrium may be approximately equal to or greater than about 4 cm. In one exemplary embodiment, the distance between the electrode 154 and 152 may be about 3 cm. In embodiments including optional electrodes 157 and 158, the distance between the electrodes 150 and 158 may be about 10 cm to about 18 cm.

Those of ordinary skill in the art appreciate that the size and spacing of the electrodes provided herein may require modification for use with patients that are larger or smaller than a typical adult and such embodiments are within the scope of the present invention. For example, smaller electrodes with a closer spacing may be required for use with a pediatric patient.

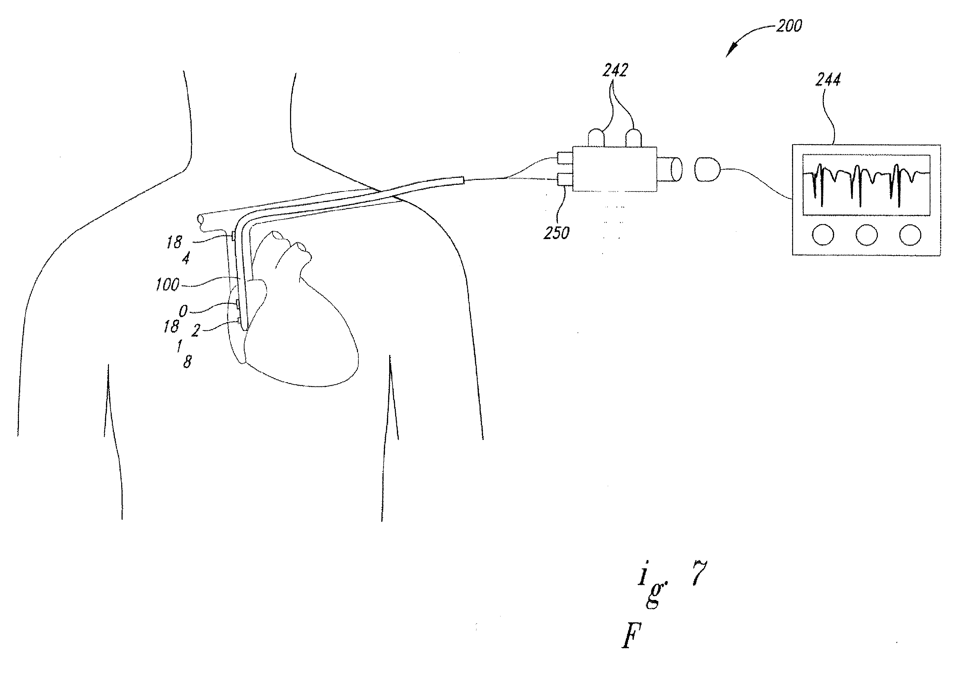

Referring to Figure 7, the CVC 100 may gain venous access to the SVC by any method known in the art including inserting the CVC 100 in a standard sterile fashion through the subclavian, one of the jugular veins, or a peripheral vein and directing the tip 112 of the CVC 100 through that vein to the SVC.

Each of the anode/cathode pairs 180 and 182 may be used to generate an ECG trace. In this manner, the ECG waveforms detected by the proximal pair 180 may be compared to the ECG waveform detected by the distal pair 182. In particular embodiments, the P wave portion of each trace is compared to determine the position of the tip 112 of the CVC 100 within the SVC, right atrium, and right ventricle.

In embodiments including the reference anode/cathode pair 184, the reference anode/cathode pair 184 may be used to generate an ECG trace. Referring to Figure 7, because the reference anode/cathode pairs 184 may be located substantially proximally from the proximal and distal anode/cathode pairs 180 and 182, the reference anode/cathode pair 184 may remain in the venous system proximal to or in the proximal SVC after the proximal and distal anode/cathode pairs 180 and 182 have entered the heart. In particular embodiments, the spacing between the anode/cathode pair 184 and the proximal pair 180 is large enough to insure the reference anode/cathode pair 184 remains proximal to or inside the proximal SVC when the distal anode/cathode pair 182 is inside the right ventricle. In this manner, the reference anode/cathode pair 184 may be used to detect the ECG waveform within venous system proximal to or in the proximal SVC while the catheter is being placed.

The ECG waveforms detected by the proximal anode/cathode pair 180 and/or distal anode/cathode pair 182 may be compared to the ECG waveform detected by the reference anode/cathode pair 184. In particular embodiments, the P wave portion of the ECG trace detected by the proximal anode/cathode pair 180 and/or distal anode/cathode pair 182 is compared to P wave portion of the ECG trace detected by the reference anode/cathode pair 184 to determine whether the tip 112 of the CVC 100 is located within the SVC, right atrium, or right ventricle.

### METHODS OF DETERMINING THE LOCATION OF THE TIP OF THE CVC USING TWO OR MORE ELECTRODE PAIRS

As is apparent to those of ordinary skill in the art, the methods 140, 450, 190, and 600 described above may be performed using the CVC 100 with two or three pairs of electrodes. With respect to each of the methods 140, 450, 190, and 600, the electrode 156 of the distal anode/cathode pair 182 may be substituted for the first electrode 114A (see Figure 3A) and the electrode 154 of the distal anode/cathode pair 182 may be substituted for the second electrode 114B (see Figure 3A). By way of another non-limiting example, any one of the electrodes 156, 154, or 152 may be used as the cathode and any one of the electrodes 154, 152, or 150 proximal to the one used as the cathode may be used as the anode. Alternatively, with respect to each of the methods 140, 450,190, and 600, one or both of the distal anode/cathode pair 182 may be substituted for the first electrode 114A (see Figure 3A) and one or both of the proximal anode/cathode pair 180 may be substituted for the second electrode 114B (see Figure 3A). However, as is appreciated by those of ordinary skill in the art, it may be desirable to use the distal most electrode as the cathode.

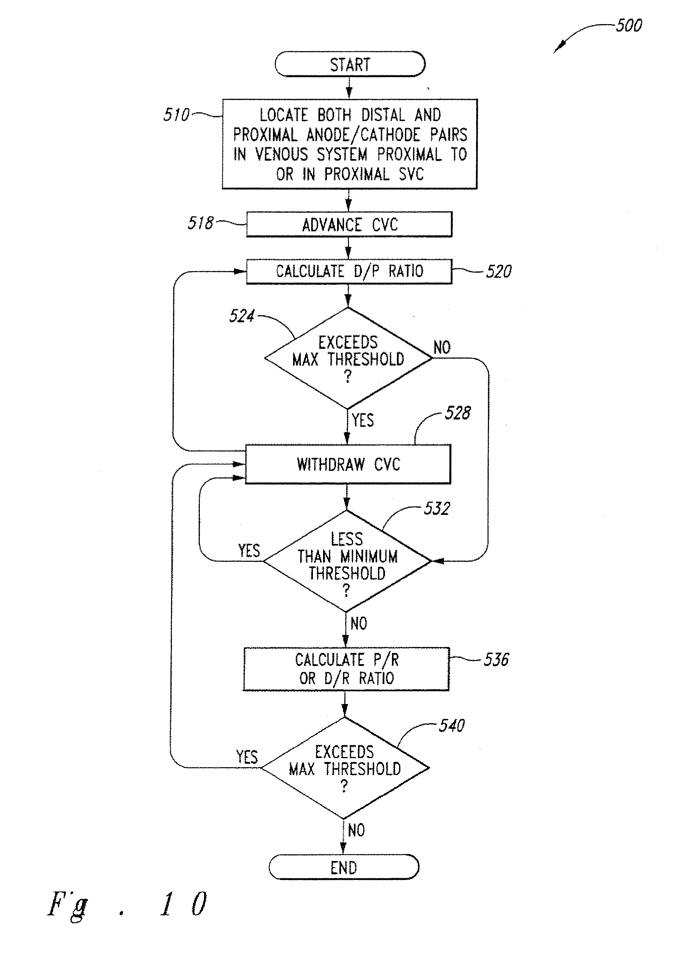

Referring to Figure 10, an alternate method 500 of determining the location of the tip 112 of the CVC 100 using two or three pairs of electrodes will now be described. With respect to method 500, unless otherwise indicated, the deflection value is calculated as the sum of the absolute value of the maximum and minimum deflections when the maximum and minimum deflections have opposite polarities. The deflection value is calculated as the larger of the absolute value of the maximum deflection and the absolute value of the minimum deflection when the maximum and minimum deflections have the same polarity.

In first block 510, both the distal anode/cathode pair 182 and the proximal anode/cathode pair 180 are located in the venous system proximal to or in the proximal SVC. A D/P ratio of the deflection value of the distal anode/cathode pair 182 to the deflection value of the proximal anode/cathode pair 180 may be calculated and used to verify the locations of the distal anode/cathode pair 182 and the proximal anode/cathode pair 180 within the venous system proximal to or in the proximal SVC. When both of the anode/cathode pairs 180 and 182 are within the venous system proximal to or in the proximal SVC, the deflection value of the P wave detected by each of them is substantially identical and the D/P ratio of their P wave deflection values equals approximately one. Optionally, the deflection value of one or both of the P waves may be stored or otherwise recorded. For example, the deflection value of the P wave detected by the distal anode/cathode pair 182 or the proximal anode/cathode pair 180 may be stored as a reference deflection value.

In next block 518, the user advances the CVC 100. By way of a non-limiting example, the user may advance the CVC 100 about 0.5 cm to about 1.0 cm. Then, in block 520, the D/P ratio of the deflection value of the distal anode/cathode pair 182 to the deflection value of the proximal anode/cathode pair 180 is calculated. Optionally, the deflection value of one or both of the P waves may be stored or otherwise recorded. Then, the method 500 advances to decision block 524.

The user or operator may wish to continue advancing the CVC 100 until the SA node is detected. When an anode/cathode pair 180 or 182 is approximately 4 cm proximal to the SA node and therefore, by inference, approximately 4 cm proximal to the entrance of the right atrium (or "caval-atrial junction," which is the location of the SA node), the deflection value of the P wave detected by that anode/cathode pair may increase.

When the distal anode/cathode pair 182 enters the right atrium and the proximal anode/cathode pair 180 is still in the venous system proximal to or in the proximal SVC, the deflection value of the P wave detected by the distal anode/cathode pair 182 may be at least four times the deflection value of the P wave detected by the proximal anode/cathode pair 180. Therefore, when the D/P ratio of the P wave deflection values of the distal anode/cathode pair 182 to the proximal anode/cathode pair 180 is greater than or equal to about 4.0 to about 8.0, the user or operator should withdraw the CVC 100. By way of a non-limiting example, the user may withdraw the CVC 100 about 0.5 cm to about 1.0 cm.

In decision block 524, a predetermined maximum threshold value "TR1" may be used to determine whether the user or operator should withdraw the CVC 100. If the D/P ratio exceeds the maximum threshold value "TR1," the decision in decision block 524 is "YES," and in block 528, the CVC 100 is withdrawn. In particular embodiments, the maximum threshold value "TR1" may range from approximately 4.0 to approximately 8.0. By way of a non-limiting example, the maximum threshold value "TR1" may be about 8.0. If the D/P ratio does not exceed the maximum threshold value "TR1," the decision in decision block 524 is "NO," and the method 500 advances to decision block 532.

When the distal anode/cathode pair 182 enters the right ventricle, the proximal anode/cathode pair 180 may be in the right atrium. Because the deflection value of the P wave experienced in the right ventricle is approximately equal to the deflection value of the P wave experienced in the proximal SVC, the D/P ratio of the P wave deflection values of the distal anode/cathode pair 182 to the proximal anode/cathode pair 180 (which is now in the upper atrium) is less than or equal to about one half. Therefore, when the D/P ratio is less than about one half, the user or operator should withdraw the CVC 100.

In decision block 532, a predetermined minimum threshold value "TMIN" may be used to determine whether the user or operator should withdraw the CVC 100. If the D/P ratio is less than the predetermined minimum threshold value "TMIN," the decision in decision block 532 is "YES," and in block 528, the CVC 100 is withdrawn. In particular embodiments, the predetermined minimum threshold value "TMIN" may be approximately one half.

If the D/P ratio is not less than the minimum threshold value "TMIN," the decision in decision block 532 is "NO," and the distal anode/cathode pair 182 and the proximal anode/cathode pair 180 may both be in the right atrium at the same time. When this occurs, the deflection value of the P waves detected by each would be very similar if not identical making their D/P ratio approximately equal to one. Therefore, in block 536, a P/R ratio or D/R ratio (described below) may be calculated to determine the location of the tip 112 of the CVC 100.

The P/R ratio may include the ratio of the deflection value of the P wave detected by the proximal anode/cathode pair 180 to the stored reference deflection value of the P wave detected in the proximal SVC. In particular embodiments, the P/R ratio may include the ratio of the deflection value of the P wave detected by the proximal anode/cathode pair 180 to a reference deflection value of the P wave detected by the reference anode/cathode pair 184. In embodiments that include a reference anode/cathode pair 184, the reference anode/cathode pair 184 may be used to detect the P wave in the proximal SVC. Because the proximal anode/cathode pair 180 is inside the right atrium, the deflection value of its P wave is greater than or equal to about four times to about eight times the deflection value of the P wave observed in the proximal SVC. When the P/R ratio is equal to or greater than a threshold value "TR2" within a range of about 4.0 to about 8.0, the user or operator should withdraw the CVC 100. By way of a non-limiting example, the threshold value "TR2" may be about 4.0. By way of a non-limiting example, the threshold value "TR2" may be equal to the predetermined maximum threshold value "TR1." Alternatively, the threshold value "TR2" could be set equal the largest D/R ratio observed thus far.

After the P/R ratio is calculated, in decision block 540, the threshold value "TR2" may be used to determine whether the user or operator should withdraw the CVC 100. If the P/R ratio exceeds the threshold value "TR2," the decision in decision block 540 is "YES," and in block 528, the CVC 100 is withdrawn. Otherwise, if the P/R ratio does not exceed the threshold value "TR2," the user does not need to withdraw the CVC 100, and the decision in decision block 540 is "NO." Then, the method 500 ends.

Alternatively, in block 536, a D/R ratio may be calculated to determine the location of the tip 112 of the CVC 100. The D/R ratio may include the ratio of the deflection value of the P wave detected by the distal anode/cathode pair 182 to the stored reference deflection value of the P wave detected in the proximal SVC. In particular embodiments, the D/R ratio may include the ratio of the deflection value of the P wave detected by the distal anode/cathode pair 182 to the reference deflection value of the P wave detected by the reference anode/cathode pair 184. In embodiments that include a reference pair 184, the reference pair 184 may be used to detect the P wave in the proximal SVC. Because the distal anode/cathode pair 182 is inside the right atrium, the deflection value of its P wave is greater than or equal to about four times to about eight times the deflection value of the P wave observed in the proximal SVC.

When D/R ratio is equal to or greater than a threshold value "TR3" within a range of about 4.0 to about 8.0, the user or operator should withdraw the CVC 100. By way of a non-limiting example, the threshold value "TR3" may be about 4.0. By way of a non-limiting example, the threshold value "TR3" may be equal to the predetermined maximum threshold value "TR1." Alternatively, the threshold value "TR3" could be set equal the largest D/R ratio observed thus far. Under these circumstances, in decision block 540, the threshold value "TR3" may be used to determine whether the user or operator should withdraw the CVC 100, i.e., if the D/R ratio exceeds the threshold value "TR3," the decision in decision block 540 is "YES," and the CVC 100 is withdrawn in block 528. Otherwise, if the D/R ratio does not exceed the threshold value "TR3," the user does not need to withdraw the CVC 100, and the decision in decision block 540 is "NO." Then, the method 500 ends.

After the CVC 100 is withdrawn in block 528, the method 500 may return to block 520 to recalculate the D/P ratio.

In method 500, determining when to withdraw the CVC 100 is unaffected by wide anatomic variability between individual people because instead of using predetermined threshold deflection values, the D/P ratio, P/R ratio, and/or D/R ratio of deflection values obtained from each individual is used.

The following table summarizes the relationship between the location of the tip 112 of the CVC 100 and the deflection values of the P waves detected by the proximal and distal anode/cathode pairs 180 and 182:

**Table 3**

| | | | | |
|---|---|---|---|---|
| Location of the distal anode/cathode pair 182 | Proximal SVC | Right Atrium | Right Atrium | Right Ventricle |
| Location of the proximal anode/cathode pair 180 | Proximal SVC | Proximal SVC | Right Atrium | Right Atrium |
| **D/P ratio:** Ratio of the deflection value of the distal anode/cathode pair 182 to the deflection value of the proximal anode/cathode pair 180 | ≈1 | ≥TR1 | ≈1 | ≤TMIN |
| **P/R ratio:** Ratio of the deflection value of the P wave detected by the proximal anode/cathode pair 180 and the deflection value of the P wave detected in the proximal SVC | ≈1 | ≈1 | ≥TR2 | ≥TR2 |
| **D/R ratio:** Ratio of the deflection value of the P wave detected by the distal anode/cathode pair 182 and the deflection value of the P wave detected in the proximal SVC | ≈1 | ≥TR3 | ≥TR3 | ≈1 |

As mentioned above, each of the threshold values "TR1," "TR2," and "TR3" in Table 3 may be within a range of about 4.0 to about 8.0 and the minimum threshold value "TMIN" may be about 0.5. Alternatively, the threshold values "TR1," "TR2," and "TR3" in Table 3 may be set equal the largest D/R ratio observed during the performance of the method 500. By way of another example, the threshold values "TR1," "TR2," and "TR3" in Table 3 may be set equal the largest D/R ratio observed for the patient during the performance of any of the methods described herein and recorded for use with the method 500. While exemplary threshold values "TR1," "TR2," "TR3," and "TMIN" have been provided for use as a general guideline, those of ordinary skill in the art appreciate that these values may benefit from adjustment as additional anatomic or electrophysiologic data is acquired and such modified values are within the scope of the present invention.

As is apparent from Table 3, either of the P/R ratio and the D/R ratio may be calculated first and used instead of the D/P ratio. For example, if the P/R ratio is calculated first, it may be compared to the threshold value "TR2." If the P/R ratio is greater than or equal to the threshold value "TR2," the tip 112 is in the right atrium or right ventricle and should be withdrawn. If the P/R ratio is less than the threshold value "TR2," the tip 112 is in the right atrium or proximal SVC. When this occurs, either the D/P ratio or the D/R ratio may be calculated. If the D/P ratio is calculated, it may be compared to the predetermined maximum threshold value "TR1." If the D/P ratio is greater than or equal to the predetermined maximum threshold value "TR1," the tip 112 should be withdrawn. If the D/R ratio is calculated, it may be compared to the threshold value "TR3." If the D/R ratio is greater than or equal to the threshold value "TR3," the tip 112 should be withdrawn.

Alternatively, if the D/R ratio is calculated first, it may be compared to the threshold value "TR3." If the D/R ratio is greater than or equal to the threshold value "TR3," the tip 112 is in the right atrium and should be withdrawn. If the D/R ratio is less than the threshold value "TR3," the tip 112 is in the right ventricle or proximal SVC. When this occurs, either the D/P ratio or the P/R ratio may be calculated. If the D/P ratio is calculated, it may be compared to the predetermined minimum threshold value "TMIN." If the D/P ratio is less than or equal to the predetermined minimum threshold value "TMIN," the tip 112 should be withdrawn. If the P/R ratio is calculated, it may be compared to the threshold value "TR2." If the P/R ratio is greater than or equal to the threshold value "TR2," the tip 112 should be withdrawn.

In addition to using the method 500 to determine when to withdraw the CVC 100, the QRS complex portion of the ECG waveforms detected by the distal anode/cathode pair 182 and/or the proximal anode/cathode pair 180 may be used to determine when the tip 112 of the CVC 100 is in the right atrium. Specifically, the tip 112 should be withdrawn because it is in the right atrium when the deflection value of the P wave detected by either the distal anode/cathode pair 182 or the proximal anode/cathode pair 180 is approximately equivalent to or greater than the voltage (or deflection value) of the QRS complex detected simultaneously by the same anode/cathode pair. The P wave and QRS complex typically look similar and deflect in the same direction. The CVC 100 may be advanced until the deflection value of the P wave is slightly less than or approximately equal to the deflection value of the QRS complex.

Further, a positive/total deflection ratio of the largest positive deflection value (of an initial positive or upwardly deflecting portion preceding a downwardly deflecting portion of a P wave detected by the distal anode/cathode pair 182 and/or the proximal anode/cathode pair 180) to the total deflection value (of the P wave detected by the distal anode/cathode pair 182 and/or the proximal anode/cathode pair 180) may be used to determine when the tip 112 of the CVC 100 is in the right atrium. As discussed above, the P wave voltage is almost entirely negative at the top of the right atrium (see trace 3 of Figure 2B), biphasic in the mid right atrium (see trace 4 of Figure 2B), and positive at the bottom of the right atrium (see trace 5 of Figure 2B). Thus, advancement of the tip 112 may be halted when the positive/total deflection ratio is greater than a predetermined fraction (e.g., one quarter, one eighth, etc.). As mentioned above, with respect to the single electrode pair embodiments, when the positive/total deflection ratio exceeds the predetermined fraction, the tip 112 is in the right atrium.

As is apparent to those of ordinary skill, the proximal and distal anode/cathode pairs 180 and 182 may be used to detect the instantaneous location of the tip 112. Therefore, if the tip 112 migrates into the atrium or ventricle, this movement may be detected immediately. Following such an occurrence, a medical professional may be alerted via a signal, such as an alarm, and the like, to reposition the tip 112.

If the tip 112 is determined to be in the atrium, the method 190 described above may be used to determine the position of the tip 112 inside the atrium. Specifically, the electrode 114B (see Figure 3A) may be attached to the skin of the patient. Then, the method 190 may be used to determine a positive/negative deflection ratio for the P wave detected by the electrode 114B and one of the electrodes 154 and 156 of the distal anode/cathode pair 182. The positive/negative deflection ratio may be compared to the first and second threshold values (see Table 2) and the location of the tip 112 within the atrium determined. Alternatively, instead of attaching the electrode 114B to the skin of the patient, one of the electrodes 157 and 158 of the reference anode/cathode pair 184 may be used. In such embodiments, the positive/negative deflection ratio is determined for the P wave detected by one of the electrodes 157 and 158 of the reference anode/cathode pair 184 and one of the electrodes 154 and 156 of the distal anode/cathode pair 182. As mentioned above, it may desirable to use the most distal electrode 156 of the distal anode/cathode pair 182. The positive/negative deflection ratio may be compared to the first and second threshold values (see Table 2) and the location of the tip 112 within the atrium determined.

Because the voltage across each of the anode/cathode pairs 180 and 182 may vary depending over time, the voltage across wires 164 and 166 and wires 160 and 162 may each constitute a time-varying signal that can be analyzed using standard signal processing methods well known in the art. In a typical patient, the maximum of voltage across the anode/cathode pairs 180 and 182 may range from about 0.2 mV to about 3 mV. The signal from each anode/cathode pairs 180 and 182 may be amplified and/or filtered to improve the signal quality. A distal signal may be detected by the distal anode/cathode pair 182 and a proximal signal may be detected by the proximal anode/cathode pair 180. Similarly, an optional reference signal may be detected by the reference anode/cathode pair 184.

A separate ECG trace may be constructed for distal and proximal signals. In some embodiments, an ECG trace may also be constructed for the reference signal. The P wave portion of one or more of these ECG traces may be identified and analyzed. For example, the ECG trace of the distal signal may be visualized by connecting wires 164 and 166 of the distal anode/cathode pair 182 to a device such as a PACERVIEW® signal conditioner designed specifically to construct and display an ECG trace from a time varying low voltage signal. Similarly, the ECG trace of the proximal signal may be viewed by connecting the wires 160 and 162 of the proximal anode/cathode pair 180 to a PACERVIEW® signal conditioner. The ECG trace of the reference signal may be viewed by connecting the wires 167 and 168 of the proximal anode/cathode pair 184 to a PACERVIEW® signal conditioner.

In particular embodiments, each of the four wires 160, 162, 164, and 166 may be coupled to a signal analysis system for analysis of the voltage information detected by the electrodes 150, 152, 154, and 156, respectively. In embodiments including electrodes 157 and 158, the wires 167 and 168 may be coupled to the signal analysis system for analysis of the voltage information detected by the electrodes 157 and 158, respectively. An exemplary signal analysis system 200 for analyzing the signals carried by wires 160, 162, 164, and 166 and alerting the user or operator when to withdraw the tip 112 of the CVC 100 may be viewed in Figure 7. In an alternate embodiment, the system 200 may also analyze the signals carried by wires 167 and 168.

### SYSTEM 200

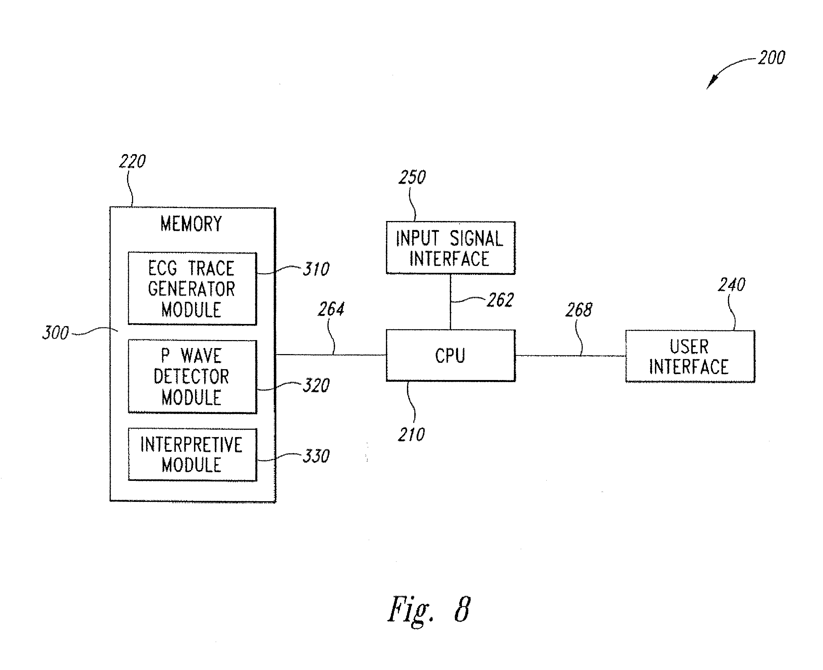

Figure 8 is a block diagram of the components of the exemplary system 200. The system 200 may include a programmable central processing unit (CPU) 210 which may be implemented by any known technology, such as a microprocessor, microcontroller, application-specific integrated circuit (ASIC), digital signal processor (DSP), or the like. The CPU 200 may be integrated into an electrical circuit, such as a conventional circuit board, that supplies power to the CPU 210. The CPU 210 may include internal memory or memory 220 may be coupled thereto. The memory 220 may be coupled to the CPU 210 by an internal bus 264.

The memory 220 may comprise random access memory (RAM) and read-only memory (ROM). The memory 220 contains instructions and data that control the operation of the CPU 210. The memory 220 may also include a basic input/output system (BIOS), which contains the basic routines that help transfer information between elements within the system 200. The present invention is not limited by the specific hardware component(s) used to implement the CPU 210 or memory 220 components of the system 200.

All or a portion of the deflection values and/or deflection ratios calculated by the methods 140, 190, 450, 500, and 600, including the reference deflection value, may be stored in the memory 220 for use by the methods.

Optionally, the memory 220 may include external or removable memory devices such as floppy disk drives and optical storage devices (e.g., CD-ROM, R/W CD-ROM, DVD, and the like). The system 200 may also include one or more I/O interfaces (not shown) such as a serial interface (e.g., RS-232, RS-432, and the like), an IEEE-488 interface, a universal serial bus (USB) interface, a parallel interface, and the like, for the communication with removable memory devices such as flash memory drives, external floppy disk drives, and the like.

The system 200 may also include a user interface 240 such as a standard computer monitor, LCD, colored lights 242 (see Figure 7), PACERVIEW® signal conditioner, ECG trace display device 244 (see Figure 7), or other visual display including a bedside display. In particular embodiments, a monitor or handheld LCD display may provide an image of a heart and a visual representation of the estimated location of the tip 112 of the CVC 100. The user interface 240 may also include an audio system capable of playing an audible signal. In particular embodiments, the user interface 240 includes a red light indicating the CVC 100 should be withdrawn and a green light indicating the CVC 100 may be advanced. In another embodiment, the user interface 240 includes an ECG trace display device 244 capable of displaying the ECG trace of the distal and proximal signals. In the embodiment depicted in Figure 7, the user interface 240 includes a pair of lights 242, one red and the other green, connected in series with a ECG trace display device 244. In some embodiments, a display driver may provide an interface between the CPU 210 and the user interface 240. Because an ultrasound machine is typically used when placing peripherally inserted central catheters ("PICC" lines), the system 200 may be incorporated into an ultrasound unit (not shown).

The user interface 240 may permit the user to enter control commands into the system 200. For example, the user may command the system 200 to store information such as the deflection value of the P wave inside the SVC. The user may also use the user interface 240 to identify which portion of the ECG trace corresponds to the P wave. The user interface 240 may also allow the user or operator to enter patient information and/or annotate the data displayed by user interface 240 and/or stored in memory 220 by the CPU 210. The user interface 240 may include a standard keyboard, mouse, track ball, buttons, touch sensitive screen, wireless user input device and the like. The user interface 240 may be coupled to the CPU 210 by an internal bus 268.

Optionally, the system 200 may also include an antenna or other signal receiving device (not shown) such as an optical sensor for receiving a command signal such as a radio frequency (RF) or optical signal from a wireless user interface device such as a remote control. The system 200 may also include software components for interpreting the command signal and executing control commands included in the command signal. These software components may be stored in memory 220.

The system 200 includes an input signal interface 250 for receiving the distal and proximal signals. The input signal interface 250 may also be configured to receive the reference signal. The input signal interface 250 may include any standard electrical interface known in the art for connecting a double dipole lead wire to a conventional circuit board as well as any components capable of communicating a low voltage time varying signal from a pair of wires through an internal bus 262 to the CPU 210. The input signal interface 250 may include hardware components such as memory as well as standard signal processing components such as an analog to digital converter, amplifiers, filters, and the like.

The various components of the system 200 may be coupled together by the internal buses 262, 264, and 268. Each of the internal buses 262, 264, and 268 may be constructed using a data bus, control bus, power bus, I/O bus, and the like.

The system 200 may include instructions 300 executable by the CPU 210 for processing and/or analyzing the distal and/or proximal signals. These instructions may include computer readable software components or modules stored in the memory 220. In particular embodiments, the instructions 300 include instructions for performing the method 500 (see Figure 10).

The instructions 300 may include an ECG Trace Generator Module 310 that generates a traditional ECG trace from the distal and/or proximal signals. In some embodiments, the ECG Trace Generator Module 310 may generate a traditional ECG trace from the reference signal. As is appreciated by those of ordinary skill in the art, generating an ECG trace from an analog signal, such as the distal and proximal signals, may require digital or analog hardware components, such as an analog to digital converter, amplifiers, filters, and the like and such embodiments are within the scope of the present invention. In particular embodiments, some or all of these components may be included in the input signal interface 250. In an alternate embodiment, some or all of these components may be implemented by software instructions included in the ECG Trace Generator Module 310. The ECG Trace Generator 310 may include any method known in the art for generating an ECG trace from a time varying voltage signal.

The ECG Trace Generator 310 may record one or more of the ECG traces generated. Presently, a chest x-ray is used to document the location of the tip 112. This documentation may be used to prove the tip 112 of the CVC was placed correctly. Using the present techniques, the recorded ECG trace(s) may be used in addition to or instead of the chest x-ray to document tip 112 location. For example, the recorded ECG trace(s) may demonstrate that the tip 112 has migrated from the proximal SVC into the right atrium. Further, the recorded ECG trace(s) could document the repositioning of the tip 112 back into proximal SVC. Additionally, the recorded ECG trace(s) could document that the tip 112 was initially placed correctly and did not migrate from its initial position. In this manner, the ECG trace(s) could be used to document the correct or incorrect placement of the tip 112 and could be included in the patient's medical record, replacing or supplementing the prior art chest x-ray. Further, if the tip 112 does migrate, the recorded ECG trace(s) could be used to determine whether the tip entered the atrium, how far into the atrium the tip migrated, and/or whether the tip entered the ventricle.

The instructions 300 may include a P Wave Detection Module 320 for detecting or identifying the P wave portion of the ECG trace. The P wave portion of the ECG trace may be detected using any method known in the art. In particular embodiments, the P Wave Detection Module 320 receives input from the user or operator via the user interface 240. The input received may identify the P wave portion of the ECG trace. Optionally, the P Wave Detection Module 320 may include instructions for identifying the QRS complex as well as the P wave portion of the ECG trace. Further, the P Wave Detection Module 320 may include instructions for determining a deflection value for an initial upwardly deflecting portion of a single P wave preceding a downwardly deflecting portion. The P Wave Detection Module 320 may also include instructions for determining a positive/total deflection ratio of the largest positive deflection value for the initial upwardly deflecting portion of the P wave to the deflection value for the entire P wave.

The instructions 300 may include an Interpretive Module 330 for comparing the P wave generated for the distal, proximal, and/or reference signals. In particular embodiments, the Interpretive Module 330 determines the deflection value of the P wave generated for the distal and/or proximal signals. In some embodiments, the Interpretive Module 330 determines the deflection value of the P wave generated for the reference signal. The Interpretive Module 330 may direct the CPU 210 to store the deflection value of the distal, proximal, and/or reference signals in memory 220. In particular, it may be desirable to store the deflection value of the P wave encountered in the proximal SVC. The Interpretive Module 330 may receive input from the user or operator via the user interface 240 instructing the Interpretive Module 330 to store the deflection value. This information could be stored under a unique patient identifier such as a medical record number so that tip location information could be accessed anytime during the life of the CVC 100, potentially avoiding the need for a chest x-ray to document current location.

With respect to performing the method 500 illustrated in Figure 10, the Interpretive Module 330 may also determine the D/P ratio by calculating the ratio of the deflection value of the distal signal to the deflection value of the proximal signal. If the D/P ratio is approximately equal to or greater than the maximum threshold value "TR1," the tip 112 of the CVC 100 may be in the right atrium. The Interpretive Module 330 may alert the user or operator that the tip 112 is in the right atrium and the CVC 100 should be withdrawn from the right atrium. On the other hand, if the D/P ratio is approximately equal to or less than the minimum threshold value "TMIN," the tip 112 of the CVC 100 may be in the right ventricle. The Interpretive Module 330 may alert the user or operator that the tip 112 is in the right ventricle and the CVC 100 should be withdrawn therefrom.

If the D/P ratio is less than the maximum threshold value "TH1" and greater than the minimum threshold value "TMIN," the tip 112 may be in either the right atrium or the proximal SVC. When this happens, the Interpretive Module 330 may calculate the P/R ratio and/or the D/R ratio. For example, if the D/R ratio is approximately equal to or greater than the threshold value "TR3," the tip may be in the right atrium and should be withdrawn therefrom. When this occurs, the Interpretive Module 330 may alert the user or operator that the tip 112 is in the right atrium. If the D/R ratio is approximately less than the threshold value"TR3," the tip 112 is in the SVC and may be advanced if the operator so chooses. The Interpretive Module 330 may communicate to the user or operator that the tip 112 may be advanced.

On the other hand, if the P/R ratio is approximately equal to or greater than the threshold value "TR2," the tip may be in the right atrium or right ventricle and should be withdrawn therefrom. When this occurs, the Interpretive Module 330 may alert the user or operator to withdraw the tip 112. If the P/R ratio is approximately less than the threshold value"TR2," the tip 112 is in the SVC (because the D/P ratio is less than the maximum threshold value "TR1") and may be advanced if the operator so chooses. The Interpretive Module 330 may communicate to the user or operator that the tip 112 may be advanced.

In an alternate embodiment, the P/R ratio is used instead of the D/P ratio. Whenever the P/R ratio is approximately equal to or greater than the threshold value "TR2," the user or operator may be alerted to withdraw the CVC 100. If the P/R ratio is approximately less than the threshold value "TR2," the D/P ratio or D/R ratio may be calculated and used to determine the position of the tip 112 of the CVC 100.

In another alternate embodiment, the D/R ratio is used instead of the D/P ratio. Whenever the D/R ratio is approximately equal to or greater than the threshold value "TR3," the user or operator may be alerted to withdraw the CVC 100. If the D/R ratio is approximately less than the threshold value "TR3," the D/R ratio or P/R ratio may be calculated and used to determine the position of the tip 112 of the CVC 100.

In particular embodiments, the instructions in the Interpretive Module 330 direct the CPU 210 to use the user interface 240 to communicate whether the tip 112 should be withdrawn to the user. The CPU 210 may use the user interface 240 to communicate the tip 112 may be advanced. Because the Interpretive Module 330 may interpret the P wave to obtain the deflection values of the distal and proximal signals, compare the deflection values, and provide the operator with immediate real-time feedback, the operator need not interpret the actual ECG waveforms.

### MONITOR 127

The monitor 127 of the system 121 for use with single pair of electrode embodiments will now be described. Returning to Figure 3A, for illustrative purposes, the monitor 127 is described as coupled to each of the electrodes 114A and 114B by wires 123 and 129, respectively. However, in alternate embodiments, the monitor 127 is coupled to the electrodes 176 and 174 of the distal anode/cathode pair 182 (see Figure 3B) by wires 123 and 129, respectively. By way of another alternate embodiment, one or both of the distal anode/cathode pair 182 may be coupled to the monitor 127 by wire 123 and one or both of the proximal anode/cathode pair 180 may be may be coupled to the monitor 127 by wire 129.

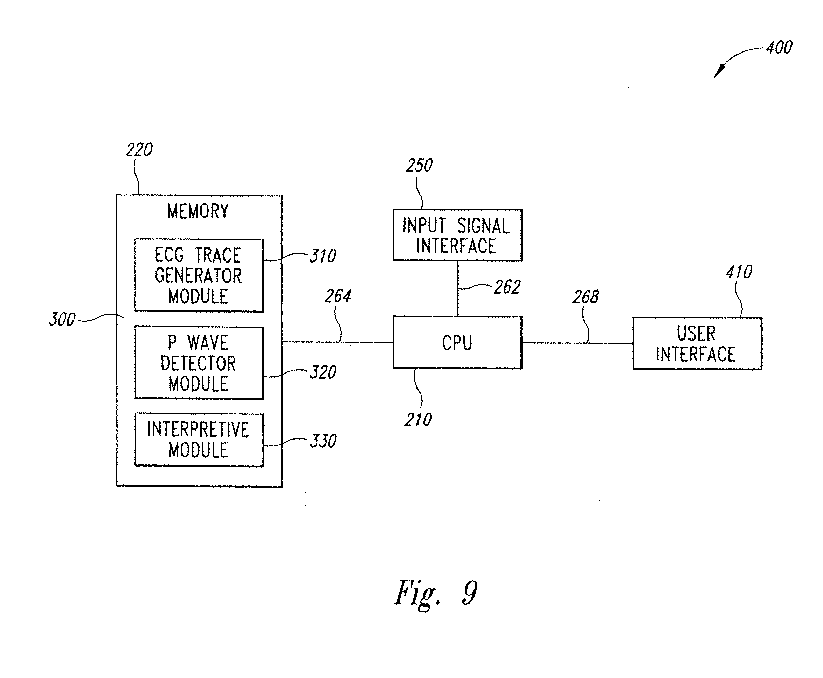

Referring to Figure 9, the monitor 127 includes a signal analysis system 400 that may be substantially similar to the signal analysis system 200 described above. Specifically, the system 400 may construct an ECG trace for the electrical signals detected by the pair of electrodes 114, and identify and analyze the P wave portion of the ECG trace using any method discussed above with respect to the system 200. Further, the system 400 is configured to display information to the user or operator communicating the current position of the tip 112 of the CVC 100.

Like reference numerals are used to identify substantially identical components in Figures 8 and 9. The system 400 includes the CPU 210, the memory 220, the input signal interface 250 for receiving the signals detected by the pair of electrodes 114, and a user interface 410. Like system 200, the various components of the system 400 may be coupled together by the internal buses 262, 264, and 268.

All or a portion of the deflection values and/or deflection ratios calculated by the methods 140, 190, 450, and 600, including the reference deflection value, may be stored in the memory 220 for use by the methods.

The system 400 differs from the system 200 with respect to the user interface 410 and at least a portion of the computer-executable instructions stored in memory 220. Returning to Figure 3A, in some embodiments, the user interface 410 includes an array of lights 412, a first portion 412A of which corresponds to ratio values below or equal to the first predefined threshold value of the method 140 (see Figure 4), a second portion 412B of which corresponds to the range of ratio values greater than the first predefined threshold value and less than or equal to the second threshold value of the method 140, and a third portion 412C of which indicates the second predefined threshold value has been exceeded.

By way of a non-limiting example, the first portion 412A may include an array of green lights. The number of green lights lit may indicate the magnitude of the ratio of the deflection value of the currently observed P wave to the reference deflection value. For example, the first portion may include eight lights. The first light may indicate the ratio is less than or equal to 0.8. For each 0.2 increase in the ratio, another green light may be lit until all eight green lights are lit and the ratio is approximately equal to 4.0. If the ratio decreases to less than 4.0, for each 0.2 decrease in the ratio, a green light may be turned off until only a single green light is lit.

By way of a non-limiting example, the second portion of lights may include an array of yellow lights. The number of yellow lights lit may indicate the magnitude of the ratio of the deflection value of the currently observed P wave to the reference deflection value. For example, the second portion may include eight yellow lights. The first yellow light may indicate the ratio is approximately equal to about 4.4 to about 4.5. For each 0.2 increase in the ratio above 4.4, another yellow light may be lit until all eight yellow lights are lit and the ratio is approximately equal to 6.0. If the ratio decreases to less than 6.0, for each 0.2 decrease in the ratio, a yellow light may be turned off until the ratio is less than 4.4 and none of the yellow lights are lit.

By way of a non-limiting example, the third portion may include a single red light indicating the magnitude of the ratio of the deflection value of the currently observed P wave to the reference deflection value has exceeded 6.0. If the ratio decreases to less than 6.0, the red light may be turned off. In this manner, the user interface 410 provides greater resolution for ratio values less than or equal to the first predefined threshold value than for ratio values between the first and second predefined threshold values.

While the above example has been described as including lights (such as LEDs, conventional light bulbs, and the like), those of ordinary skill in the art appreciate that any indicator may used and such embodiments are within the scope of the present teachings. For example, a monitor displaying a graphical representation of lights, a figure, such as a bar, that increases or decreases in size to reflect an increase or decrease in the ratio, and the like may be used in place of the array of lights. Optionally, the user interface 410 may include a screen 420 (see Figure 3A) configured to display information to the user or operator. For example, the screen 420 may display an image of a heart and a visual representation of the estimated location of the tip 112 of the CVC 100. Alternatively, the screen 420 may display the words "ADVANCE," "HOLD," or 'WITHDRAW" as appropriate.

The user interface 410 may also indicate when the tip 112 is located in the ventricle. By way of a non-limiting example, the user interface 410 may include a light, audio signal, or other indicator configured to indicate the tip 112 has entered the ventricle.

In some embodiments, a display driver may provide an interface between the CPU 210 and the user interface 410. Optionally, the user interface 410 includes an audio system capable of playing an audible signal. For example, the audio system may produce a tone that increases in pitch as the ratio increases and decreases in pitch as the ratio decreases may be used. Alternatively, the audio system may produce one tone when the ratio is greater than the first predefined threshold value, indicating the tip 112 should be withdrawn. In such embodiments, the audio system may produce another more urgent or annoying tone when the ratio is above the second predefined threshold value. Further, the audio system may be silent when the ratio is below the first predefined threshold value.

The user interface 410 may permit the user to enter control commands into the system 200. For example, the user may command the system 200 to store information such as the deflection value of the P wave inside the proximal SVC (e.g., the reference deflection value), atrium (e.g., the reference atrium deflection value), and the like. The user may also use the user interface 410 to identify manually which portion of the ECG trace corresponds to the P wave. The user interface 410 may also allow the user or operator to enter patient information (such as a patient identifier number) and/or annotate the data displayed by user interface 410 and/or stored in memory 220 by the CPU 210. The user interface 410 may include a standard keyboard, mouse, track ball, buttons 416, touch sensitive screen, wireless user input device and the like. The user interface 410 may be coupled to the CPU 210 by an internal bus 268.

By way of a non-limiting example, a patient's reference deflection value (detected when the tip 112 was located in the proximal SVC) and/or reference atrium deflection value (detected when the tip 112 was located in the atrium) could be stored in the memory 220 and associated with the patient's patient identifier number or some other identification number. In this manner, the patient's recorded reference deflection value could be used anytime during the life of the CVC 100 by recalling the reference deflection value from memory 220 using the patient's patient identifier number, which could be entered manually using the user interface 410.

Optionally, the system 400 may also include an antenna or other signal receiving device (not shown) such as an optical sensor for receiving a command signal such as a radio frequency (RF) or optical signal from a wireless user interface device such as a remote control. The system 400 may also include software components for interpreting the command signal and executing control commands included in the command signal. These software components may be stored in memory 220.

The system 400 includes instructions 300 executable by the CPU 210 for processing and/or analyzing the electrical signals detected by the pair of electrodes 114. The instructions 300 may include the ECG Trace Generator Module 310 that generates a traditional ECG trace from the signals detected by the pair of electrodes 114, the P Wave Detection Module 320 for detecting or identifying the P wave portion of the ECG trace, and the Interpretive Module 330. As discussed above with respect to the system 200, the ECG Trace Generator Module 310 may record the ECG trace generated thereby.

Optionally, the P Wave Detection Module 320 may include instructions for identifying the QRS complex as well as the P wave portion of the ECG trace. Further, the P Wave Detection Module 320 may include instructions for determining a deflection value for an initial upwardly deflecting portion of a single P wave preceding a downwardly deflecting portion. The P Wave Detection Module 320 may also include instructions for determining a positive/total deflection ratio of the largest positive deflection value for the initial upwardly deflecting portion of the P wave to the deflection value for the entire P wave.

Like the Interpretive Module 330 of the system 200, the Interpretive Module 330 of the system 400 determines the deflection value of the P wave detected by a pair of electrodes (i.e., the pair of electrodes 114). However, other functions performed by the Interpretive Module 330 may differ in system 400 from those performed by the Interpretive Module 330 in system 200. The Interpretive Module 330 directs the CPU 210 to store the deflection value of the P wave detected by the pair of electrodes 114 in the proximal SVC in memory 220. The Interpretive Module 330 may receive input from the user or operator via the user interface 410 instructing the Interpretive Module 330 to store the deflection value.

The Interpretive Module 330 also includes instructions for performing the method 140, 190, and/or the method 600. With respect to the method 140 (see Figure 4), the Interpretive Module 330 may determine the ratio of the deflection value of the currently observed P wave to the reference deflection value. The Interpretive Module 330 may compare this ratio to the first predefined threshold (e.g., about 1.5). If the ratio is less than or equal to the first predefined threshold, the Interpretive Module 330 determines the tip 112 is in the SVC. Further, if the determination is made in block 143, the Interpretive Module 330 may signal the user or operator to advance the tip 112. If the determination is made in block 145, the Interpretive Module 330 may signal the user or operator to stop withdrawing the tip 112.

If the ratio is greater than the first predefined threshold, the Interpretive Module 330 determines the tip 112 is not in a desired location and instructs the user interface 410 to signal the user or operator to withdraw the tip.

The Interpretive Module 330 may compare the ratio to the second predefined threshold value (e.g., about 2.0). If the ratio is less than or equal to the second predefined threshold value, the Interpretive Module 330 determines the tip 112 is in the distal SVC near the SA node or in the atrium near the SA node. If the ratio is greater than the second predefined threshold value, the Interpretive Module 330 determines the tip 112 is in the atrium.

Optionally, the Interpretive Module 330 may determine the ratio of the deflection value of the current P wave detected by the pair of electrodes 114 to the reference atrium deflection value. The Interpretive Module 330 may compare this ratio to the third predefined threshold value (e.g., about 0.5) to determine whether the tip 112 is in the atrium or the ventricle.

With respect to the method 190 (see Figure 6), the Interpretive Module 330 may determine the tip 112 is located within the right atrium using any method known in the art or disclosed herein. The Interpretive Module 330 then calculates the positive/negative deflection ratio and compares this ratio to the first predetermined threshold value (e.g., about 0.30). If the positive/negative deflection ratio is less than the first predetermined threshold value, the Interpretive Module 330 determines the tip 112 is in the upper right atrium. On the other hand, if the positive/negative deflection ratio is greater than or equal to the first predetermined threshold value, the Interpretive Module 330 compares positive/negative deflection ratio to the second predetermined threshold value (e.g., about 1.30). If the positive/negative deflection ratio is greater than the second predetermined threshold value, the Interpretive Module 330 determines the tip 112 is in the lower atrium. Otherwise, if the positive/negative deflection ratio is greater than or equal to the first predetermined threshold value and less than or equal to the second predetermined threshold value, the Interpretive Module 330 determines the tip 112 is in the mid atrium.

With respect to the method 600 (see Figures 11A and 11B), the Interpretive Module 330 may store a reference deflection value and determine the deflection ratio of the deflection value of the currently observed P wave to the reference deflection value. After the tip 112 is withdrawn or advanced, the Interpretive Module 330 may determine the current deflection ratio of the deflection value of the currently observed P wave to the reference deflection value. The deflection ratio determined before the tip 112 was withdrawn or advanced is stored by the Interpretive Module 330 as a previous deflection ratio.

Each time the tip 112 is moved (e.g., advanced or withdrawn), the Interpretive Module 330 determines whether the current deflection ratio is the largest observed since the CVC 100 was inserted into the venous system, and if the current deflection ratio is the largest deflection ratio observed, stores the current deflection ratio as the maximum deflection ratio.

The Interpretive Module 330 compares the current deflection ratio to the previous deflection ratio. If the current deflection ratio is less than or equal to the previous deflection ratio, the Interpretive Module 330 determines the tip 112 has been advanced too far and may signal the user to withdraw the CVC 100. Then, the Interpretive Module 330 determines whether the tip 112 has been withdrawn far enough. The Interpretive Module 330 may determine the tip 112 has been withdrawn far enough when the positive/total deflection ratio is less than a predetermined fraction (e.g., one quarter, one eighth, etc.). Otherwise, the tip 112 has not been withdrawn far enough. If the tip 112 has not been withdrawn far enough, the Interpretive Module 330 signals the user to withdraw the tip 112.

If the tip 112 has been withdrawn far enough, the Interpretive Module 330 determines whether the current deflection ratio is approximately equal to the maximum deflection ratio. If the current deflection ratio is not approximately equal to the maximum deflection ratio, the Interpretive Module 330 determines the tip 112 has been withdrawn too far and signals the user to advance the CVC 100.

As mentioned above, the Interpretive Module 330 may compare current deflection ratio to the previous deflection ratio. If the current deflection ratio is greater than the previous deflection ratio, the Interpretive Module 330 determines whether the current deflection ratio is less than a maximum threshold value (e.g., 8.0). If the current deflection ratio is less than the maximum threshold value, the Interpretive Module 330 determines the tip 112 has not been advanced far enough and signals the user to advance the CVC 100.

If instead the current deflection ratio is greater than or equal to the maximum threshold value, the Interpretive Module 330 determines whether the current deflection ratio is approximately equal to the maximum threshold value. If the current deflection ratio is not approximately equal to the maximum threshold value, the Interpretive Module 330 determines the tip 112 has been advanced too far and signals the user to withdraw the CVC 100. After the tip 112 is withdrawn, the Interpretive Module 330 determines whether the current deflection ratio is approximately equal to the maximum threshold value.

If the current deflection ratio is not approximately equal to the maximum threshold value, the Interpretive Module 330 determines whether the current deflection ratio is less than the maximum threshold value (indicating the tip 112 has been withdrawn too far). If the current deflection ratio is less than the maximum threshold value, the Interpretive Module 330 determines the tip 112 has been withdrawn too far and signals the user to advance the CVC 100. On the other hand, if the current deflection ratio is still greater than the maximum threshold value, the Interpretive Module 330 determines the tip 112 has not been withdrawn far enough and signals the user to withdraw the CVC 100.

Optionally, whenever the current deflection ratio is approximately equal to the maximum deflection ratio or the maximum threshold value, the Interpretive Module 330 may signal the user to stop moving (e.g., advancing or withdrawing) the CVC 100.

With respect to the methods 140, 190, and 600, the instructions in the Interpretive Module 330 direct the CPU 210 to use the user interface 410 to communicate whether the tip 112 should be withdrawn to the user. Further, the instructions in the Interpretive Module 330 direct the CPU 210 to use the user interface 410 to communicate the tip 112 may be advanced. Because the Interpretive Module 330 may interpret the P wave to obtain the deflection values, compare the deflection values, and provide the operator with immediate real-time feedback, the operator need not interpret the actual ECG waveforms.

Optionally, the system 400 could be coupled to a prior art navigational system, such as a VIASYS MedSystems NAVIGATØR® BioNavigation® system, manufactured by VIASYS Healthcare Inc. of Conshohocken, Pennsylvania, which is principally used to guide peripherally placed lines, such as a peripherally inserted central catheter ("PICC"). Such systems determine the location of a PICC line using magnetic fields that are generated by a detector, and detected by a magnetically activated position sensor located in the tip of a stylet inserted inside the PICC near its tip. By way of a non-limiting example, the detector may include a NAVIGATØR® electronic locating instrument configured to emit low-level, high-frequency magnetic fields. Also by way of a non-limiting example, the stylet may include a MAPCath® Sensor Stylet also manufactured by VIASYS Healthcare inc.

The sensor in the tip of the stylet is activated by the magnetic field emitted by the detector. The sensor is operable to output its location via a wire coupled to the sensor and extending longitudinally along the PICC to the detector, which is configured to interpret the signal and communicate the location of the tip to the user or operator. As mentioned above, because such navigational systems depend upon a relationship between surface landmarks and anatomic locations, they cannot be used to determine the location of the tip 112 of the CVC 100 with sufficient accuracy. However, the system 400 may be used, in conjunction with a navigational system to improve the accuracy of the navigational system.

By way of non-limiting examples, additional prior art navigational systems include the Sherlock Tip Location System used with the stylet provided in Bard Access Systems PICC kits both of which are manufactured by C. R. Bard, Inc. of Salt Lake City, Utah, the CathRite™ system manufactured by Micronix Pty. Ltd. of Australia, and the like. As is apparent to those of ordinary skill in the art, the present teaching may be combined with one or more of these additional exemplary navigational systems and used to improve the accuracy of those systems.

In further embodiments, the teachings provided herein may be expanded to use electrophysiology procedures for navigating other bodily channels. For example, in the prior art, ECG guidance techniques have been used to place feeding tubes. Because the QRS complex measured in the stomach is upright but in a post pyloric area (first part of the duodenum, beyond the stomach) the QRS complex is down-going, one or more threshold deflection values may be determined and used to determine the placement of the tip of the feeding tube.

The foregoing described embodiments depict different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected", or "operably coupled", to each other to achieve the desired functionality.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention. Furthermore, it is to be understood that the invention is solely defined by the appended claims. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation *is* explicitly recited, those skilled in the art will recognize that such recitation should typically be interpreted to mean *at least* the recited number (e.g., the bare recitation of "two recitations," without other modifiers, typically means *at least* two recitations, or *two or more* recitations).

Accordingly, the invention is not limited except as by the appended claims.

In the following, there is listed numbered items of the present disclosure corresponding to the claims as originally filed of the parent application, EP 09 707 467.8.
1. A method of locating a tip of a central venous catheter, the central venous catheter having a portion disposed within the superior vena cava, a distal pair of electrodes, and a proximal pair of electrodes spaced longitudinally from the distal pair of electrodes, the method comprising:
   obtaining a distal electrical signal from the distal pair of electrodes;
   generating a first P wave from the distal signal;
   determining a first deflection value of the first P wave;
   obtaining a proximal electrical signal from the proximal pair of electrodes;
   generating a second P wave from the proximal signal;
   determining a second deflection value of the second P wave;
   calculating a first ratio from the first and second deflection values; and
   determining a location of the tip of the central venous catheter based on the first ratio.
2. The method of 1, wherein determining the location of the tip of the central venous catheter based on the first ratio comprises:
   comparing the first ratio to a maximum threshold value; and
   concluding the tip of the central venous catheter is located in the right atrium if the first ratio exceeds the maximum threshold value.
3. The method of 2, wherein the maximum threshold value has a value between about 4.0 and about 8.0.
4. The method of 1, wherein determining a location of the tip of the central venous catheter based on the first ratio comprises:
   comparing the first ratio to a minimum threshold value; and
   concluding the tip of the central venous catheter is located in the right ventricle if the first ratio is less than the minimum threshold value.
5. The method of 4, wherein the minimum threshold value has a value of about 0.5.
6. The method of 1, wherein determining a location of the tip of the central venous catheter based on the first ratio comprises:
   comparing the first ratio to a maximum threshold value and a minimum threshold value; and
   concluding the tip is located in one of the superior vena cava and the right atrium if the first ratio is less than the maximum threshold value and greater than the minimum threshold value.
7. The method of 1, wherein the portion of the central venous catheter disposed within the superior vena cava has a reference pair of electrodes spaced longitudinally proximally from the proximal pair of electrodes, and the method further comprises:
   obtaining a reference electrical signal from the reference pair of electrodes;
   generating a SVC P wave from the reference signal;
   determining a SVC deflection value of the SVC P wave;
   calculating a second ratio of one of the first and second deflection values to the SVC deflection value; and
   determining a second location of the tip of the central venous catheter based on the second ratio.
8. The method of 7, wherein determining the second location of the tip of the central venous catheter based on the second ratio comprises:
   comparing the second ratio to a maximum threshold value; and
   concluding the tip of the central venous catheter is located in the right atrium if the second ratio exceeds the maximum threshold value.
9. The method of 1, comprising:
   obtaining a SVC deflection value;
   calculating a second ratio of one of the first and second deflection values to the SVC deflection value; and
   determining a second location of the tip of the central venous catheter based on the second ratio.
10. The method of 9, wherein determining the second location of the tip of the central venous catheter based on the second ratio comprises:
   comparing the second ratio to a maximum threshold value; and
   concluding the tip of the central venous catheter is located in the right atrium if the second ratio exceeds the maximum threshold value.
11. The method of 9, wherein determining the SVC deflection value comprises:
   positioning the distal pair of electrodes inside the superior vena cava;
   obtaining a SVC electrical signal from the distal pair of electrodes;
   generating a SVC P wave from the SVC signal; and
   calculating the SVC deflection value from the SVC P wave.
12. The method of 9, wherein determining the SVC deflection value comprises:
   positioning the proximal pair of electrodes inside the superior vena cava;
   obtaining a SVC electrical signal from the proximal pair of electrodes;
   generating a SVC P wave from the SVC signal; and
   calculating the SVC deflection value from the SVC P wave.
13. The method of 1, wherein the first P wave comprises a maximum value and a minimum value, each having a polarity,
   determining the first deflection value of the first P wave comprises determining the larger of the absolute value of the maximum value and the absolute value of the minimum value when the maximum value and the minimum value have the same polarity; and
   determining the first deflection value of the first P wave comprises totaling the absolute values of the maximum value and the minimum value when the maximum and minimum values have opposite polarities.
14. A method of locating a tip of a central venous catheter, the central venous catheter having a portion disposed within the superior vena cava; a distal pair of electrodes; and a proximal pair of electrodes spaced longitudinally from the distal pair of electrodes, the method comprising:
   obtaining a distal electrical signal from the distal pair of electrodes;
   generating a first P wave from the distal signal;
   obtaining a proximal electrical signal from the proximal pair of electrodes;
   generating a second P wave from the proximal signal;
   comparing the first and second P waves; and
   determining a location of the tip of the central venous catheter based on the comparison of the first and second P waves.
15. The method of 14, wherein the first P wave has a first shape and the second P wave has a second shape and comparing the first and second P waves comprises comparing the first and second shapes.
16. The method of 15, wherein the first shape has a first height and the second shape has a second height and determining the location of the tip of the central venous catheter based on the comparison of the first and second shapes comprises concluding the tip of the central venous catheter is located in the right atrium if the first height is at least four times larger than the second height.
17. The method of 15, wherein the first shape has a first height and the second shape has a second height and determining the location of the tip of the central venous catheter based on the comparison of the first and second shapes comprises concluding the tip of the central venous catheter is located in the right ventricle if the first height is at most half the second height.
18. The method of 15, wherein the first shape has a first height and the second shape has a second height and determining the location of the tip of the central venous catheter based on the comparison of the first and second shapes comprises concluding the tip of the central venous catheter is located in either the right atrium or superior vena cava if the first height is approximately equal to the second height.
19. The method of 14, wherein the portion of the central venous catheter disposed within the superior vena cava has a reference pair of electrodes, the method comprising:
   obtaining a reference electrical signal from the reference pair of electrodes;
   generating a reference P wave from the reference signal; and
   determining a second location of the tip of the central venous catheter based on the comparison of the first and reference P waves.
20. The method of 19, wherein the first P wave has a first shape and the reference P wave has a reference shape and comparing the first and reference P waves comprises comparing the first and reference shapes.
21. The method of 20, wherein determining the second location of the tip of the central venous catheter based on the comparison of the first and reference shapes comprises concluding the tip of the central venous catheter is located in the right ventricle if the first shape approximates a mirror image of the reference shape about a horizontal axis.
22. The method of 19, wherein the first shape has a first height and the reference shape has a reference height and determining the second location of the tip of the central venous catheter based on the comparison of the first and reference shapes comprises concluding the tip of the central venous catheter is located in the right atrium if the first height is at least four times larger than the reference height.
23. The method of 19, wherein the first shape has a first height and the reference shape has a reference height and determining the second location of the tip of the central venous catheter based on the comparison of the first and reference shapes comprises concluding the tip of the central venous catheter is located in either the superior vena cava or right ventricle if the first height is approximately equal to the reference height.
24. The method of 14, wherein the portion of the central venous catheter disposed within the superior vena cava has a reference pair of electrodes, the method comprising:
   obtaining a reference electrical signal from the reference pair of electrodes;
   generating a reference P wave from the reference signal; and
   determining a second location of the tip of the central venous catheter based on the comparison of the second and reference P waves.
25. The method of 24, wherein the second P wave has a second shape and the second shape has a second height and the reference P wave has a reference shape and the reference shape has a reference height and determining the second location of the tip of the central venous catheter based on the comparison of the second and reference P waves comprises concluding the tip of the central venous
   catheter is located in the right atrium if the second height is at least four time larger than the reference height.
26. A computer-readable medium having computer executable instructions for determining the location of a tip of a central venous catheter based on a first P wave detected by a first pair of electrodes disposed near the tip of the central venous catheter and a second P wave detected by a second pair of electrodes disposed on the central venous catheter and spaced proximally from the first pair, the instructions comprising:
   calculating a first deflection value as a function of the first P wave;
   calculating a second deflection value as a function of the second P wave;
   comparing the first deflection value to the second deflection value; and
   determining a location of the tip of the central venous catheter based on the comparison of the first and second deflection values.
27. The computer-readable medium of 26, wherein the instructions for comparing the first and second deflection values comprise instructions for calculating a first ratio of the first deflection value to the second deflection value and the instructions for determining the location of the tip of the central venous catheter based on the comparison of the first and second deflection values comprise instructions for comparing the first ratio to a predetermined maximum threshold.
28. The computer-readable medium of 26, wherein the instructions for comparing the first and second deflection values comprise instructions for calculating a first ratio of the first deflection value to the second deflection value and the instructions for determining the location of the tip of the central venous catheter based on the comparison of the first and second deflection values comprise instructions for comparing the first ratio to a predetermined minimum threshold.
29. The computer-readable medium of 26, comprising instructions for obtaining a reference deflection value, comparing one of the first and second deflection values to the reference deflection value, and determining a second location of a tip of the central venous catheter based on the comparison of the one of the first and second deflection values to the reference deflection value.
30. The computer-readable medium of 29, wherein the instructions for comparing one of the first and second deflection values to the reference deflection value comprise instructions for calculating a second ratio of one of the first and second deflection values to the reference deflection value and instructions for determining the second location of the tip of the central venous catheter based on the comparison of one of the first and second deflection values to the reference deflection value comprise instructions for comparing the second ratio to a predetermined maximum threshold.
31. The computer-readable medium of 26, wherein the first and second P waves comprise a series of discrete numerical values and the instructions for calculating the deflection value as a function of the P wave comprise instructions for
   determining the maximum value within the series of discrete numerical values of the P wave;
   determining the minimum value within the series of discrete numerical values of the P wave;
   determining the polarities of the maximum and minimum values; comparing the polarity of the maximum value to the polarity of the minimum value;
   if the polarity of the maximum value is the same as the polarity of the minimum value, determining the larger of the absolute value of the maximum value and the absolute value of the minimum value; and
   if the polarity of the maximum value is not the same as the polarity of the minimum value, totaling the absolute values of the maximum value and the minimum value.
32. A method of determining a location of a tip of a central venous catheter having a lumen filed with an electrolytic material, a portion disposed inside the superior vena cava, and a first electrode in communication with the electrolytic material, the tip having an opening in communication with the lumen that exposes the electrolytic material inside the lumen to the electrical environment outside the tip, the method comprising:
   attaching a second electrode to the skin of a subject;
   positioning the tip of the central venous catheter within the superior vena cava;
   while the tip is positioned within the superior vena cava, obtaining a reference electrical signal comprising the voltage difference between the first electrode and the second electrode;
   generating a reference P wave from the reference electrical signal;
   determining a reference deflection value of the reference P wave;
   one of advancing and withdrawing the tip of the central venous catheter to a new position;
   while the tip is in the new position, obtaining a new electrical signal comprising the voltage difference between the first electrode and the second electrode;
   generating a new P wave from the new electrical signal;
   determining a new deflection value of the new P wave;
   calculating a ratio of the new deflection value to the reference deflection value;
   comparing the ratio to a threshold value; and
   one of advancing or withdrawing the tip based on the comparison of the ratio to the threshold value.
33. The method of 32, wherein the one of advancing or withdrawing the tip based on the comparison of the ratio to the threshold value comprises:
   advancing the tip if the ratio is less than the threshold value; and
   withdrawing the tip if the ratio is greater than the threshold value.
34. The method of 32, wherein the threshold value is less than a ratio of a deflection value of a P wave detectable near the sino-atrial node to the reference deflection value.
35. The method of 32, further comprising:
   if the ratio is equal to the threshold value, maintaining the tip of the central venous catheter in its current position.
36. The method of 32, further comprising:
   if the ratio is greater than the threshold value, comparing the ratio to a second threshold value; and
   if the ratio is greater than the second threshold value, determining the tip of the central venous catheter is in the right atrium and withdrawing the tip.
37. A method of determining a location of a tip of a central venous catheter having a lumen filed with an electrolytic material, a portion disposed inside the superior vena cava, a second electrode attached to the skin of a subject, and a first electrode in communication with the electrolytic material, the tip having an opening in communication with the lumen that exposes the electrolytic material inside the lumen to the electrical environment outside the tip, the method comprising:
   obtaining a reference electrical signal comprising the voltage difference between the first electrode and the second electrode;
   generating a reference P wave from the reference electrical signal;
   determining a reference deflection value of the reference P wave;
   after the tip of the central venous catheter has been moved to a new position, obtaining a new electrical signal comprising the voltage difference between the first electrode and the second electrode;
   generating a new P wave from the new electrical signal;
   determining a new deflection value of the new P wave;
   calculating a ratio of the new deflection value to the reference deflection value;
   comparing the ratio to a threshold value; and
   indicating the tip should be withdrawn based on the comparison of the ratio to the threshold value.
38. A method of determining in which portion of the atrium a tip of a central venous catheter is located, the method comprising:
   obtaining an electrical signal from a portion of the atrium adjacent the tip of the central venous catheter;
   generating a P wave from the electrical signal;
   determining a greatest positive deflection value for the reference P wave;
   determining a smallest negative deflection value for the reference P wave;
   calculating a ratio of the greatest positive deflection value to the smallest negative deflection value;
   comparing the ratio to a first threshold value;
   if the ratio is less than the first threshold value, determining the tip is in the upper atrium;
   comparing the ratio to a second threshold value;
   if the ratio is greater than the second threshold value, determining the tip is in the lower atrium; and
   if the ratio is greater than or equal to the first threshold value, and the ratio is less than or equal to the second threshold value, determining the tip is in the mid atrium.
39. A computer-readable medium storing computer executable instructions for directing a processor to:
   obtain a reference deflection value from a reference electrical signal, detected near a tip of a central venous catheter when the tip was located in a first position inside the superior vena cava;
   obtain a new deflection value from a new electrical signal detected near the tip of a central venous catheter after the tip was relocated from the first position to a second position;
   calculate a ratio of the new deflection value to the reference deflection value;
   compare the ratio to a threshold value;
   if the ratio is less than the threshold value, signal to a user the tip may be advanced; and
   if the ratio is greater than the threshold value, signal to the user to withdraw the tip.
40. A method of determining a location of a tip of a central venous catheter having an electrode disposed inside the venous system of a subject, the venous system of the subject comprising a superior vena cava having a proximal portion near a sino-atrial node, the method comprising:
   positioning the tip of the central venous catheter within the venous system of the subject proximal to or within a proximal portion of the superior vena cava;
   while the tip is positioned proximal to or within a proximal portion of the superior vena cava, generating a reference P wave using the electrode;
   determining a reference deflection value of the reference P wave;
   one of advancing and withdrawing the tip of the central venous catheter to a first position;
   while the tip is in the first position, generating a first P wave using the electrode;
   determining a first deflection value of the first P wave;
   calculating a first ratio of the first deflection value to the reference deflection value;
   one of advancing and withdrawing the tip of the central venous catheter to a second position;
   while the tip is in the second position, generating a second P wave using the electrode;
   determining a second deflection value of the second P wave;
   calculating a second ratio of the second deflection value to the reference deflection value;
   comparing the first ratio to the second ratio;
   if the second ratio is less than the first ratio, withdrawing the tip of the central venous catheter until a P wave generated using the electrode has a minimum negative deflection value that is greater than a predetermined portion of the second deflection value of the second P wave; and
   if the second ratio is greater than the first ratio, determining whether the second ratio exceeds a maximum threshold value, if the second ratio does not exceed the maximum threshold value, advancing the tip of the central venous catheter and if the second ratio exceeds the maximum threshold value, withdrawing the tip of the central venous catheter.
41. The method of 40, further comprising:
   after each time the tip of the central venous catheter is advanced or withdrawn, generating a new P wave using the electrode, determining a new deflection value of the new P wave, and determining a new ratio of the new deflection value to the reference deflection value;
   identifying a maximum ratio using the new ratio determined after each time the tip of the central venous catheter is advanced or withdrawn;
   if the second ratio is less than the first ratio, after the tip of the central venous catheter has been withdrawn until the minimum negative deflection value is greater than the predetermined portion of the second deflection value of the second P wave, the tip is located in a current location, generating a current P wave using the electrode, determining a current deflection value of the current P wave, and determining a current ratio of the current deflection value to the reference deflection value; and
   determining whether the current ratio is approximately equal to the maximum ratio and if the current ratio is approximately equal to the maximum ratio, maintaining the tip of the central venous catheter in its present location.
42. The method of 41, further comprising:
   if the current ratio is not approximately equal to the maximum ratio, advancing the tip of the central venous catheter.
43. The method of 40, wherein the predetermined portion of the P wave is greater than three quarters of the total P wave.
44. The method of 40, wherein the predetermined portion of the P wave is greater than seven eighths of the total P wave.
45. The method of 40, further comprising:
   if the second ratio is approximately equal to the maximum threshold value, maintaining the tip of the central venous catheter in its present location.
46. The method of 40, further comprising:
   after each time the tip of the central venous catheter is advanced or withdrawn, generating a new P wave using the electrode, determining a new deflection value of the new P wave, and determining a new ratio of the new deflection value to the reference deflection value;
   identifying a maximum ratio using the new ratio determined after each time the tip of the central venous catheter is advanced or withdrawn;
   one of advancing and withdrawing the tip to a current location;
   generating a current P wave using the electrode;
   determining a current deflection value of the current P wave;
   determining a current ratio of the current deflection value to the reference deflection value; and
   determining whether the current ratio is approximately equal to the maximum ratio and if the current ratio is approximately equal to the maximum ratio, maintaining the tip of the central venous catheter in its present location.
47. A method of determining a location of a tip of a central venous catheter having an electrode disposed inside the venous system of a subject, the venous system of the subject comprising a superior vena cava having a proximal portion near a sino-atrial node, the method comprising:
   at least one of advancing and withdrawing the tip the central venous catheter;
   after each advancement or withdrawal of the tip of the central venous catheter, generating a new P wave using the electrode, determining a new deflection value of the new P wave, and determining a new ratio of the new deflection value to the reference deflection value;
   identifying a maximum ratio using the new ratio determined after each advancement or withdrawal of the tip of the central venous catheter;
   one of advancing and withdrawing the tip to a current location;
   generating a current P wave using the electrode;
   determining a current deflection value of the current P wave;
   determining a current ratio of the current deflection value to the reference deflection value;
   determining whether the current P wave has a minimum negative deflection value that is greater than a predetermined portion of the current P wave; and
   determining whether the current ratio is approximately equal to the maximum ratio and if the current ratio is approximately equal to the maximum ratio, maintaining the tip of the central venous catheter in its present location.
48. The method of 47, further comprising:
   determining whether the current ratio exceeds a maximum threshold value, if the current ratio does not exceed the maximum threshold value, advancing the tip of the central venous catheter and if the current ratio exceeds the maximum threshold value, withdrawing the tip of the central venous catheter.
49. A monitor electrically couplable to an electrode attached to a tip of a central venous catheter that is insertable into a venous system of a subject, the monitor comprising:
   a processor;
   a withdraw indicator coupled to the processor configured to indicate when the tip of the central venous catheter should be withdrawn; and
   a memory coupled to the processor and having instructions executable by the processor, when executed, the instructions instructing the processor to:
      store an identifier identifying the subject,
      determine and store a reference deflection value of a P wave detected by the electrode attached to the tip of the central venous catheter,
      associate the reference deflection value with the identifier identifying the subject,
      determine a current deflection value of a current P wave detected by the electrode attached to the tip of the central venous catheter,
      calculate a current ratio of the current deflection value to the stored reference deflection value, and
      actuate the withdraw indicator to indicate the tip of the central venous catheter should be withdrawn based on the current ratio.
50. The monitor of 49, wherein the instructions executable by the processor further instruct the processor to:
   store a maximum ratio comprising a largest ratio of the current deflection value to the stored reference deflection value calculated for the subject,
   associate the maximum ratio with the identifier identifying the subject,
   compare the current ratio to the maximum ratio, and
   actuate the withdraw indicator to indicate the tip of the central venous catheter should be withdrawn based on the comparison.
51. The monitor of 49, wherein the instructions executable by the processor further instruct the processor to compare the current ratio to a threshold value and actuate the withdraw indicator to indicate the tip of the central venous catheter should be withdrawn based on the comparison.
52. The monitor of 49, wherein the instructions executable by the processor further instruct the processor to store at least one of the current deflection value and the current ratio.

## Claims

1. A device, comprising:
a central venous catheter having a lumen fillable with an electrolytic material, a portion disposable inside the superior vena cava, and a first electrode arranged for communication with the electrolytic material, the central venous catheter comprising a tip having an opening in communication with the lumen for exposing the electrolytic material inside the lumen to the electrical environment outside the tip, the device further comprising:
a second electrode for attaching to the skin of a subject;
the tip of the central venous catheter positionable within the superior vena cava; the device configured for:
while the tip is positioned within the superior vena cava, obtaining a reference electrical signal comprising the voltage difference between the first electrode and the second electrode;
generating a reference P wave from the reference electrical signal; and
determining a reference deflection value of the reference P wave;
the tip of the central venous catheter for one of advancing and withdrawing t to a new position; the device configured for:
while the tip is in the new position, obtaining a new electrical signal comprising the voltage difference between the first electrode and the second electrode;
generating a new P wave from the new electrical signal;
determining a new deflection value of the new P wave;
calculating a ratio of the new deflection value to the reference deflection value; comparing the ratio to a threshold value;
wherein the tip is one of advanceable or withdrawable based on the comparison of the ratio to the threshold value.

2. The device of claim 1, wherein the threshold value is less than a ratio of a deflection value of a P wave detectable near the sino-atrial node to the reference deflection value.

3. The device of claim 1 or 2, further comprising: if the ratio is greater than the threshold value, comparing the ratio to a second threshold value; and if the ratio is greater than the second threshold value, determining the tip of the central venous catheter is in the right atrium, the tip being and withdrawable.

4. A device, comprising:
a central venous catheter having a lumen fillable with an electrolytic material, a portion disposable inside the superior vena cava, a second electrode attachable to the skin of a subject, and a first electrode for communication with the electrolytic material, the central venous catheter comprising a tip having an opening in communication with the lumen that exposes the electrolytic material inside the lumen to the electrical environment outside the tip, the device configured for:
obtaining a reference electrical signal comprising the voltage difference between the first electrode and the second electrode;
generating a reference P wave from the reference electrical signal;
determining a reference deflection value of the reference P wave;
after the tip of the central venous catheter has been moved to a new position, obtaining a new electrical signal comprising the voltage difference between the first electrode and the second electrode;
generating a new P wave from the new electrical signal; determining a new deflection value of the new P wave;
calculating a ratio of the new deflection value to the reference deflection value;
comparing the ratio to a threshold value; and
indicating the tip should be withdrawn based on the comparison of the ratio to the threshold value.

5. A system for determining a location of a tip of a central venous catheter, the system comprising:
a central venous catheter having a lumen fillable with an electrolytic material, a portion disposable inside the superior vena cava, and a first electrode for communication with the electrolytic material, the central venous catheter having a tip having an opening in communication with the lumen that exposes the electrolytic material inside the lumen to the electrical environment outside the tip; the system comprising:
a second electrode attachable to the skin of a subject;
the tip of the central venous catheter positionable within the superior vena cava;
the system comprising a computer readable instructions for directing a processor to:
while the tip is positioned within the superior vena cava, obtain a reference electrical signal comprising the voltage difference between the first electrode and the second electrode;
generate a reference P wave from the reference electrical signal;
determine a reference deflection value of the reference P wave;
indicate one of advancing and withdrawing the tip of the central venous catheter to a new position;
while the tip is in the new position, obtain a new electrical signal comprising the voltage difference between the first electrode and the second electrode;
generate a new P wave from the new electrical signal;
determine a new deflection value of the new P wave;
calculate a ratio of the new deflection value to the reference deflection value;
comparing the ratio to a threshold value; and
indicate one of advancing or withdrawing the tip based on the comparison of the ratio to the threshold value.

6. The system of claim 5, wherein the the instruction directing the processor to indicate one of advancing or withdrawing the tip based on the comparison of the ratio to the threshold value comprise:
instructions to indicate advancing the tip if the ratio is less than the threshold value; and
instructions to indicate withdrawing the tip if the ratio is greater than the threshold value.

7. The system of claim 5, wherein the threshold value is less than a ratio of a deflection value of a P wave detectable near the sino-atrial node to the reference deflection value.

8. The system of claim 5, the instructions directing the processor so that:
if the ratio is equal to the threshold value, provide an indication to maintain the tip of the central venous catheter in its current position.

9. The method of claim 5, the instructions directing the processor so that:
if the ratio is greater than the threshold value, the ratio is compared to a second threshold value; and if the ratio is greater than the second threshold value, determining the tip of the central venous catheter is determined to be in the right atrium and an indication is issued to with the tip.

10. A system for of determining a location of a tip of a central venous catheter, the system comprising:
a central venous catheter having a lumen fillable with an electrolytic material, a portion disposable inside the superior vena cava, a second electrode attachable to the skin of a subject, and a first electrode for communication with the electrolytic material, the central venous catheter having a tip having an opening in communication with the lumen that exposes the electrolytic material inside the lumen to the electrical environment outside the tip, the system comprising:
computer readable instructions for directing a processor to:
obtain a reference electrical signal comprising the voltage difference between the first electrode and the second electrode;
generate a reference P wave from the reference electrical signal;
determine a reference deflection value of the reference P wave; after the tip of the central venous catheter has been moved to a new position, obtain a new electrical signal comprising the voltage difference between the first electrode and the second electrode;
generate a new P wave from the new electrical signal;
determine a new deflection value of the new P wave;
calculate a ratio of the new deflection value to the reference deflection value;
compare the ratio to a threshold value; and
indicate the tip should be withdrawn based on the comparison of the ratio to the threshold value.

11. The system of any one of claims 5 to 10, comprising a user interface for said indication(s).
